Europäisches Patentamt

European Patent Office

Office européen des brevets

(19)

(11) **EP 0 648 495 B1**

(12) ## EUROPEAN PATENT SPECIFICATION

(45) Date of publication and mention
of the grant of the patent:
**05.01.2000 Bulletin 2000/01**

(51) Int. Cl.[7]: **A61K 31/715**, A61K 9/10,
A61K 38/00, A23L 1/308,
A23L 1/0524, A23L 1/0526

(21) Application number: **94111053.8**

(22) Date of filing: **15.07.1994**

(54) **Cation-complexed polysaccharides**

Kation-complexierte Polysaccharide

Polysaccharides complexés pour des cations

(84) Designated Contracting States:
**AT BE CH DE DK ES FR GB IT LI NL PT SE**

(30) Priority: **16.07.1993 US 93231**

(43) Date of publication of application:
**19.04.1995 Bulletin 1995/16**

(73) Proprietor:
**HERCULES INCORPORATED
Wilmington, Delaware 19894-0001 (US)**

(72) Inventors:
• **Barnum, Paquita Erazo
Newark, Delaware 19711 (US)**
• **Majewicz, Thomas George
Kenneth Square, Pennsylvania 19348 (US)**

(74) Representative:
**Hansen, Bernd, Dr. Dipl.-Chem. et al
Hoffmann Eitle,
Patent- und Rechtsanwälte,
Arabellastrasse 4
81925 München (DE)**

(56) References cited:
| | |
|---|---|
| **EP-A- 0 029 724** | **EP-A- 0 040 048** |
| **EP-A- 0 191 572** | **EP-A- 0 483 070** |
| **EP-A- 0 506 563** | **WO-A-87/04350** |
| **WO-A-91/04674** | **WO-A-93/19613** |
| **WO-A-93/25096** | **FR-A- 2 073 254** |
| **FR-M- 7 686** | **US-A- 3 474 086** |
| **US-A- 5 104 677** | |

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

## Description

Background of Invention

This invention is related to improved ingestible liquids. More specifically, this invention relates to such liquids that provide a low viscosity composition which among other things is useful in normalizing bowel function in individuals consuming such compositions. In addition, this invention relates to novel cation-complexed polysaccharides, methods for making the same and their use in the above described ingestible liquids.

Nutritional therapy (including use as nutritionals or pharmaceuticals) is a means for improving and/or maintaining the nutritional and health status of both normal and medically compromised mammals, including humans. It is well known that the administration of liquid nutritional therapy to mammals is often accompanied by various gastrointestinal disturbances referred to as intolerance. The most prevalent of these is irregular bowel function such as constipation and diarrhea. Diarrhea can lead to fluid or electrolyte imbalance and malnutrition. Abnormal bowel function would include unbalanced nutrient and water absorption which could result in constipated or diarrheal responses. Various means have been recommended for normalizing gastrointestinal function and reducing diarrhea disturbances associated with liquid nutritional therapy. These include pharmacological, surgical and nutritional means. However, diarrheal disturbances are still a common side effect accompanying liquid nutritional therapy despite the use of any of these means to reduce diarrheal disturbances.

A nutritional means for normalizing gastrointestinal function includes the enrichment of liquid nutritionals with plant derived dietary fiber. Numerous types of dietary fibers are currently available. Basically, dietary fiber passes through the small intestine undigested by enzymes and is a kind of natural and necessary laxative. Dietary fiber is a term that is well understood by those skilled in the art and includes nonstarch polysaccharides and lignin that are not digested by the enzymatic secretions of the gastrointestinal tract. Examples of these fibers are celluloses, hemicelluloses, pectin, gums, mucilages, lignin and lignin material varying in different plants according to type and age. Plant derived dietary fiber compositions which have been suggested for enrichment of liquid nutritionals include four classes of fiber differentiable by their solubility in water and fermentability by colonic microflora. These classes of fiber described in the prior art include soluble-fermentable, soluble-nonfermentable, insoluble-fermentable and insoluble-nonfermentable.

Dietary fibers of the water insoluble and fermentable type include soy polysaccharide which has been employed in nutritional liquids. However, recent studies indicate that soy polysaccharide fiber enriched liquid nutritionals produce a statistically insignificant reduction of diarrheal disturbances as compared to fiber free liquid nutritionals. The investigators stated, "in this study the fiber supplementation had only a small, nonsignificant effect on diarrhea." (Tube Feeding - Related Diarrhea in Acutely Ill Patients:, by P.A. Guenter et al., The Journal of Parenteral and Enteral Nutrition; Vol. 15, No. 3, pp. 277-280 (1991)).

Dietary fibers of the type that are both water insoluble and non-fermentable include pea fiber, corn bran fiber and oat hull fiber. These fibers have been suggested for normalizing bowel function, particularly in combination with dietary fibers from one of the other groupings. For example, a commercially available fiber enriched liquid nutritional called Ultracal® is marketed by Mead-Johnson Nutritionals, a division of Bristol-Myers Squibb, Evansville, Indiana and contains a combination of soy polysaccharide and oat hull fibers.

Dietary fibers of the type that are water soluble and non-fermentable include all cellulosic derivatives such as carboxymethyl derivatives which have been suggested also to be useful in combination with fibers from one of the other groupings of dietary fibers for normalizing bowel function.

Dietary fibers of the type that are water soluble and fermentable include pectin, psyllium, acacia, carrageenan, guar, locust bean and others.

Carrageenan is a dietary fiber that is used in most commercial enteral products as a colloidal emulsion stabilizing agent.

Water-soluble pectin has been shown to reverse diarrheal disturbances in healthy humans when added to a fiber free liquid nutritional. As used herein, the term "pectin" may include one or more polygalacturonates selected from the group consisting of pectins and pectates. Pectin is also intended to include pectic acids, pectinic acids and pectinates. Pectin has also been shown to significantly enhance intestinal adaptation to massive small bowel resection in laboratory animals. However, enrichment of liquid nutritionals with pectin has not been commercially successful due to a number of practical considerations. Liquid nutritionals are typically sterilized by various heat methods such as pasteurization and autoclave treatments. Prior art pectin enriched liquid nutritionals have exhibited rheological and thermodynamic instability following such heat treatments. "Rheological instability" refers to an unacceptable increase in the viscosity of the liquid, including gelation of a continuous phase. "Thermodynamic instability" refers to precipitation or coagulation of the components in the liquid leading to an unstable dispersion. The pectin that has been employed has been water soluble and the combination of such pectin with the proteins, peptides, and amino acids found in liquid nutritionals has exacerbated the increase in viscosity. This problem is intensified when liquid nutritionals are administered via infusion through a fine bore delivery device such as a nasogastral tube when it becomes very important that the flow of nutrients not be restricted due to high viscosity of the liquid or by the precipitation or coagulation of components in the liquid. In prior art fiber enriched nutritional liquids this is a particular problem after sterilization which results in a significant increase in the viscosity of the liquid. Another factor is that in the event of oral consumption of the liquid nutritional, it must be pleasing to the palate and non-gritty. It was previously stated that "It is virtually impossible to formulate a good tasting, high fiber drink using insoluble forms of fiber." --Food Technology, January 1987, pp. 74-75.

A number of publications describe the attempts that have been made to introduce added dietary fiber into nutritional drinks and other beverages. In U.S. patent 4,988,530 a combination of gum arabic and soluble pectin was added to fruit and vegetable beverages. The gum arabic was added due to its minimal effect upon beverage viscosity. However, gum arabic could not be used alone since the required concentration levels would exceed allowable Food and Drug Administration limits. The high-methoxyl pectins utilized in this patent had a degree of esterification greater than 50% and preferably greater than 70% and were employed under conditions of concentration and soluble solids level and pH that do not induce gelation.

U.S. Patent 4,959,227 discloses non-fat milk liquid foods containing dietary fibers including both water-insoluble and soluble types of dietary fiber. The water insoluble types of fiber are cellulose fiber and the water soluble fibers are plant gums and plant gum derivatives such as locust bean gum, citrus pectin, low and high methoxy pectin, gum tragacanth, agar, carrageenan, xanthan gum, guar gum, and alginate.

In European Patent Application publication number 0 483 070 A2 by Greenberg is disclosed a nutritionally complete feeding composition containing hydrolysed soluble fiber, especially guar gum or pectin.

In U.S. Patent 4,198,400 is disclosed the addition of protopectin and a cellulose type of dietary fiber to a water-reconstituted juice or soup composition wherein the cellulose type fiber is coated to render it more hydrophilic and improve mouthfeel.

U.S. Patent 5,104,677 discloses a liquid nutritional product comprising a fat source and a dietary fiber system.

WO-A-93/25096 discloses psyllium drink mix compositions containing psyllium husks and edible water-soluble salts. The salts are present at a level sufficient to reduce the gellation rate of the psyllium husk when dispersed in an aqueous solution.

WO-A-93/19613 discloses a gelling system as a fat substitute. A substance with a cellulosic or saccharide-derived backbone is used in salt form in the composition. This particular mixture is then emulsified in oil. Particular examples of polysaccharides used include pectates and alginates.

US-A-3 474 086 discloses organic derivatives of bismuth and aluminium and a process for their production. The derivatives comprise pectin.

EP-A-0 506 563 discloses a suspension of alginic acid, sodium bicarbonate and optionally magnesium alginate. The composition is indicated to be useful as a pharmaceutical.

FR-A-2 073 254 discloses stable homogenous compositions constituting guar gum and at least one therapeutically active substance which is insoluble in water. Barium salts are excluded. Tetrabasic phytate salts with aluminium may also be present in the composition.

EP-A-0 029 724 describes quick-setting low methoxyl pectin compositions. The pectin is provided with a slowly dissolving calcium ion source and a slowly dissolving edible acid as well as a solubilising amount of sugar.

FR-M-7 686 describes a medicine for the treatment of gastro-intestinal problems. A composition is disclosed containing aluminium pectate suspended in water.

EP-A-0 191 572 discloses a method in which a gelled skin is formed around foodstuff by bringing together gellable material with a gelling activator. A possible gellable material is indicated to be a non-toxic alkali metal alginate.

WO-A-91/04674 discloses a gelling system. Alginate and pectate gels are produced from an aqueous mixture of water-soluble or water-dispersible alginate and pectinate and a sparingly soluble calcium ion source. The mixture is heated to induce gellation.

WO-A-87/04350 discloses a process for improving the adherence of gels to mucous membranes. Two components capable of forming a gel are applied to the same area of a mucous membrane (such as a metallic salt and at least one polysaccharide). Alginic acid and pectin are mentioned as possible polysaccharides.

EP-A-0 040 048 discloses calcium alginate fibres. The fibres are indicated to be insoluble and produced by the reaction of algin in a first aqueous solution with bivalent or tervalent metal cations in a second aqueous solution. The resulting fibres are indicated to be stable to heat.

Prior to the present invention there remained a need for dietary fiber, particularly for use in nutritional liquids, such as enteral formulations, that reduced the incidence of diarrheal disturbances associated with the administration of such liquids. Prior art has demonstrated the efficacy of water-soluble pectin in such formulations in normal humans and laboratory animals, but its practical use was obviated by considerations of viscosity and stability of the formulation.

It has not been suggested previously that cation-complexed mineral enriched dietary fibers, including calcium-complexed pectins, would be useful for liquid nutritionals or for liquid nutritional therapy. Instead it has been stated that pectin had not been successfully applied in enteral formulations for technical reasons, since these compositions underwent extreme product thickening or coagulation after the required heat treatment. It also has not been suggested that mineral enriched fibers, including calcium-complexed pectin would be useful for improving the gastrointestinal function of mammals including human adults, children and infants who have had part of their gastrointestinal tract removed. The present invention concerns liquid nutritionals which are useful for improving the gastrointestinal function of normal and medically compromised mammals, including human adults, children and infants. These liquid nutritionals are also useful for reducing diarrheal disturbances associated with the therapeutic administration of liquid nutritionals to mammals, including humans as well as reducing the diarrheal disturbances associated with a physical condition such as a surgically shortened bowel. In such cases, it is difficult for the individual to efficiently absorb nutrients and water and diarrheal disturbances commonly accompany the intake of solid or liquid nutritionals under these circumstances.

Accordingly, the present invention concerns dietary fiber enriched liquid nutritionals which are useful for improving the nutritional status and health of both normal and medically compromised mammals, including human adults, children, and infants when administered *ad libitum* or by direct infusion into the gastrointestinal system through an enteral feeding tube. The amount of fiber added to a feeding composition may vary depending on the needs of the person and whether the composition is to be taken orally or enterally. Thus the fiber content of the composition may vary according to the amount of composition intended to be ingested per day. It has been found that adding a water-insoluble, cation-complexed anionic polysaccharide fiber to an otherwise nutritionally complete liquid food composition containing peptides, proteins or amino acids can control diarrhea and improve gastrointestinal function while retaining the low viscosity character of the food composition.

Thus, the present invention provides a substantially water-insoluble, cation-complexed anionic polysaccharide, said cation being selected from alkaline earth metal cations, alkali metal cations, transition metal cations, protein, amino acids, oligopeptides, cationic or amino-functionalized polysaccharides, cationic or amino-functionalized synthetic polymers, or combinations thereof, wherein said cation-complexed anionic polysaccharide comprises particles in a dry, agglomerated form that readily disperse and disaggregate when added to an aqueous medium to liberate smaller particles having a median particle size less than 100 μm. These polysaccharides have antidiarrheal efficacy in humans. The present invention also provides an ingestible composition comprising the substantially water-insoluble, cation-complexed anionic polysaccharides of the present invention dispersed in an aqueous medium, wherein said ingestible composition has a low viscosity and is heat sterilizable. The water-insoluble, cation-complexed anionic polysaccharides of the present invention lack, or make an insignificant, contribution to beverage viscosity, flavor, color or coagulation during or after heat treatments commonly used for product sterilization. Furthermore, the polysaccharides cause no or minor increases to the viscosity of such liquids that contain at least one of proteins, peptides and amino acids.

The polysaccharides and compositions of the present invention can be administered in an aqueous antidiarrheal composition to an animal, especially to humans. The polysaccharides and compositions of the invention can also be used in a method of treating weight loss or diarrhea in an animal, including humans, comprising enterally administering to said animal a therapeutically effective amount of a water insoluble cation-complexed anionic polysaccharide. Such treatment is especially useful wherein said animal has had a part of its gastrointestinal tract removed by a surgical procedure. The compositions are useful as a nutritional means of improving gastrointestinal function including bowel function, increasing absorption of nutrients and water by the gastrointestinal tract and for reducing diarrheal disturbances due to abnormal bowel function.

Another aspect of the present invention provides a method of preparing the substantially water-insoluble, heat sterilizable, cation-complexed anionic polysaccharide of the present invention comprising wetting a substantially water-soluble anionic polysaccharide with water to a total solids level of about 30 to 70%, adding a source of cations followed by mixing in a high intensity mixer to a total solids content of about 30 to 70%.

In another aspect, this invention provides a low viscosity, heat sterilizable feeding composition comprising: carbohydrates providing approximately 1 to 99% of total calories; protein, peptides and amino acids providing approximately 1 to 99% of total calories; lipid containing essential fatty acids providing approximately 1 to 99% of total calories; vitamins; minerals; water; and at least one substantially water-insoluble, cation-complexed anionic polysaccharide of the present invention. In a preferred embodiment carbohydrates provide 20-85% of total calaries; proteins, peptides or amino acids provide 10-30% of total calories and lipid containing fatty acids provide 2-50% of total calories. This same composition may be prepared in a dehydrated state and then subsequently rehydrated when ready for use.

The cations that are complexed with the polysaccharides are metal ions, proteins, peptides, amino acids, oligopeptides, cationic or amino-functionalized polysaccharides, cationic or amino-functionalized synthetic polymers, and mixtures thereof. The cation complexed with the polysaccharides is preferably a metal ion derived from salts selected from the group consisting of alkaline earth metal salts, alkali metal salts and transition metal salts and combinations thereof. The preferred salts are salts of calcium, magnesium, zinc or iron. As used herein, the term "salt" includes inorganic salts, inorganic oxides, inorganic hydroxides and organic salts. When a metal ion is used as the cation, it is preferably selected from the group consisting of calcium, iron, magnesium, zinc, potassium, sodium, copper, and manganese and mixtures thereof. More preferably the metal cations are selected from the group consisting of calcium, iron, zinc and magnesium. Most preferably the cation is calcium. Mixtures of two or more metal cations may be employed. However, if a monovalent metal cation is employed, a divalent metal cation, such as calcium, is preferably present. Preferably when such mixtures are used, one of the metal cations is calcium. Examples of metal salts that can be used in the practice of the present invention include, but are not limited to, barium sulfate, bismuth salicylate, calcium acetate, calcium acid phosphate, calcium adipate, calcium alanate, calcium ascorbate, calcium-DL-asparagine, calcium aspartate, calcium benzoate, DL-calcium bimalate, calcium butyrate, calcium iso-butyrate, calcium caproate, calcium carbonate, calcium caseinate, calcium cellobionate, calcium chloride, calcium citrate, calcium dihydrogen phosphate, calcium formate, calcium fumarate, calcium galactonate, calcium glucoheptonate, calcium gluconate, calcium glutamate, calcium glutaminate, calcium glycerate, calcium glycerophosphate, calcium glycinate, calcium glycolate, calcium heptoate, calcium hexanoate, calcium hydrogen phosphate, calcium hydroxide, calcium iodide, calcium-2-keto-D-gluconate, calcium-5-keto-D-gluconate, calcium lactate, calcium lactophosphate, calcium laurate, calcium levulinate, calcium magnesium carbonate, calcium magnesium inositol hexaphosphate, calcium malate, calcium maleate, calcium malonate, calcium methionate, calcium octoate, calcium oleate, calcium oxalate, calcium oxide, calcium palmitate, calcium phosphate tribasic, calcium-o-phosphate, calcium 3-phospho-D-glycerate, calcium phosphoryl choline chloride, calcium propionate, calcium pyrophosphate, calcium-D-saccharate, calcium sorbate, calcium succinate, calcium sucrate, calcium sulfite, calcium tartrate, calcium sulfonate, calcium tetraphosphate, calcium sulfate, calcium thiosulfate, calcium trihydroxygluterate, calcium tryptophanate, iron (II) acetate, iron (III) acetate, iron (III) acetate hydroxide, iron alluminate, iron (III) ammonium chloride, iron (III) ammonium citrate, iron (II) ammonium sulfate, iron (II) L-aspartate, iron (III) benzoate, iron (II) carbonate, iron (II) carbonate saccharate, iron (II) chloride, iron (III) chloride, iron choline citrate, iron (III) choline citrate, iron (III) choline stearate, iron (II) citrate, iron dextran, iron (II) formate, iron (III) formate, iron (II) fumarate, iron (II) gluconate, iron (III) glycerophosphate, iron (III) hypophosphite, iron (II) lactate, iron maleate, iron (II) octoate, iron (III) octoate, iron (III) oleate, iron (II) oxalate, iron (II) oxalate dihydrate, iron (III) oxalate, iron (II) phosphate, iron (III) potassium oxalate, iron (III) potassium tartrate, iron (III) pyrophosphate, iron (III) sodium citrate, iron (III) sodium oxalate, iron (III) sodium pyrophosphate, iron succinate, iron (II) sulfate, iron (III) sulfate, iron (II) D-tartrate, iron (III) tartrate, iron (III) valerate, magnesium acetate, magnesium acetylsalicylate, magnesium adipate, magnesium ammonium phosphate, magnesium ammonium sulfate, magnesium ascorbate, magnesium aspartate, magnesium benzoate, magnesium butyrate, magnesium carbonate, magnesium chloride, magnesium citrate, magnesium dihydrogen phosphate, magnesium formate, magnesium fumarate, magnesium glucoheptonate, magnesium gluconate, magnesium-L-glutamate, magnesium glycerophosphate, magnesium glycinate, magnesium hydrogen phosphate, magnesium hydrogen-o-phosphate, magnesium hydroxide, magnesium hydroxide carbonate, magnesium lactate, magnesium laurate, magnesium malate, magnesium maleate, magnesium malonate, magnesium mandelate, magnesium nitrate, magnesium oleate, magnesium oxalate, magnesium oxide, magnesium phosphate, magnesium propionate, magnesium pyrophosphate, magnesium succinate, magnesium sulfate, magnesium sulfonate, magnesium tartrate, magnesium tryptophanate, zinc acetate, zinc adipate, zinc ammonium chloride, zinc ammonium sulfate, zinc ascorbate, zinc aspartate, zinc-iso-butyrate, zinc calcium bromide, zinc carbonate, zinc chloride, zinc citrate, zinc formate, zinc fumarate, zinc glucoheptonate, zinc gluconate, zinc glycerophosphate, zinc glycinate, zinc heptanoate, zinc heptoate, zinc hydrogen phosphate, zinc hydroxide, zinc lactate, zinc laurate, zinc linoleate, zinc malate, zinc maleate, zinc nitrate, zinc octoate, zinc oleate, zinc oxide, zinc palmitate, zinc phosphate, zinc phosphate monobasic, zinc phosphate tribasic, zinc-o-phosphate, zinc propionate, zinc pyrophosphate, zinc pyruvate, zinc resinate, zinc saccharinate, zinc sorbate, zinc succinate, zinc sulfate, zinc tartrate, zinc tryptophanate, zinc tyrosinate, zinc valerate, and zinc-iso-valerate. The preferred salts are calcium salts such as calcium chloride, calcium hydroxide, calcium acetate, calcium propionate, calcium oxide, calcium gluconate, calcium lactate, calcium carbonate, and calcium sulfate.

Examples of proteins, peptides, oligopeptides, amino acids, and glycoproteins, that may be used as cations include, but are not limited to gelatin, casein, albumin, whey, soy, substance P, tachykinins, vasoactive intestinal polypeptide, growth hormone, serotonin, gastrin, insulin, enterogastrin, gastrin-releasing peptide, pancreatozymin, parathormone, bombesin, secretin, cholecystokinin, pancreatozymin, somatostatin, thyrotropin-releasing factor, motilin, neurotensin, galanin, peptide YY, pentagastrin, gastric inhibitory polypeptide, arginine, glutamine, leucine, histidine, alanine, lysine, tyrosine, phenylalanine, tryptophan, L-alanyl-L-glutamine, N-acetyl-L-glutamine, alanyl-L-tyrosine, glycly-tyrosine, glycyl-1-glutamine, immunoglobulins, antibodies, and lactoferrin. The chemical or enzymatic hydrolysis products of these proteins may also be employed in the practice of the present invention. In most instances, the proteins, peptides, oligopeptides, amino acids and glycoproteins are used only in combination with one or more of the metal cations. Preferably, at least one of the one or more metal cations used in combination with the protein is calcium. Examples of cationic or amino-functionalized polysaccharides are chitin, chitosan, cationic guar, and cationic starch. Examples of cationic or amino-functionalized synthetic polymers are cholestyramine, polyethyleneimine, polyoxazolines, polyamidoamines, and cationic polyacrylates or cationic polyacrylamides. These cationic or amino-functionalized polymers may be used in combination with one or more metal cations.

The water-insoluble, cation-complexed, anionic polysaccharide is derived from anionic polysaccharides that are carboxylated, sulfated, or phosphated. Most preferred are the carboxylated polysaccharides such as alginates, pectins, arabic, karaya, tragacanth, ghatti, psyllium, flax seed, okra, xanthan, gellan, carboxymethylcellulose, carboxymethylguar, carboxymethylstarch and hyaluronic acid. Examples of sulfated polysaccharides are carrageenan, chondroitin sulfate, dermatan sulfate, heparan sulfate, keratan sulfate, keratan, heparin, and cellulose sulfate. Examples of phosphated polysaccharides are phosphated cellulose, yeast mannan, phosphated glucan and phosphated starch. More generally any anionic polysaccharide or blends of anionic polysaccharides that can be insolubilized with a cationic reagent may be useful. Blends of anionic polysaccharides with neutral polysaccharides such as guar, starch, inulin, hydroxypropylcellulose, methylcellulose, or methylhydroxypropylcellulose, locust bean, dextrans, dextrin, scleroglucan, pullulan, curdlan, ethyl cellulose, or synthetic polymers such as poly(vinyl alcohol), poly(vinylpyrrolidone), poly(ethylene oxide), and poly(propylene glycol) may also be useful.

In the anionic polysaccharides used as starting materials in the practice of the present invention, the cation counterion, if present, is preferably sodium, potassium or hydrogen. The preferred anionic polysaccharides are derived from pectin polysaccharides. The preferred pectin polysaccharides have a degree of methyl or acetyl esterification less than about 45%, preferably less than about 30%, more preferably less than about 15% and most preferably less than about 5%. Some pectins, such assugar beet pectin, may also have acetyl groups. The degree of polymerization ( DP) of the pectin polysaccharides is preferably at a minimum of about 50 and more preferably greater than about 150. The DP values correspond to molecular weights of the pectin polysaccharides preferably at a minimum of 8800 daltons and more preferably greater than 26,400 daltons as determined by relative viscosity measurements. Other anionic polysaccharides have similar molecular weight criteria.

The low viscosity ingestible compositions of the present invention, depending upon the application may have a viscosity of 1,000 cps or less, preferably 500 cps or less, and more preferably 100 cps or less, measured at a shear rate of about 100 $sec^{-1}$. In some applications, the viscosity is 35 cps or less. In preferred applications the viscosity is 100 cps or less. These viscosity levels are found in the ingestible liquids both before and after heat treatments such as pasteurization or sterilization. The lower levels of viscosity are preferred in liquids administered to the patient through a tube, while higher levels of viscosity are acceptable in certain orally consumed beverages. In any event in enteral liquids administered through a tube, the viscosity must be at a level which permits the patient to receive sufficient nutrition and which permits unobstructed flow through the tube.

The product of complexation of the cation with the anionic polysaccharide is substantially water-insoluble, and provides minimal increase in viscosity to the ingestible compositions even after heat sterilization. The increase is less than 1000 cps, preferably less than 100 cps, and most preferably less than 25 cps.

The preferred compositions are prepared from the pectin polysaccharides and calcium salts. The level of calcium salt necessary to form the water-insoluble complexes of this invention is dependent on the pectin composition and the nature of the calcium salt. The molar ratio of the calcium added to the anhydrogalacturonic acid in the pectin is generally between 0.05 to 1.5. At the preferred molar ratios of 0.1 to 0.6 calcium to anhydrogalacturonic acid, the degree of water insolubility of the cation-complexed anionic polysaccharide is greater than about 70 percent.

The cation-complexed anionic polysaccharide is substantially water-insoluble. In general, at least 50% of the cation-complexed anionic polysaccharide is insoluble in water. Preferably, at least 60% of the cation-complexed anionic polysaccharide is insoluble in water. More preferably, at least 70% of said cation-complexed anionic polysaccharide is insoluble in water. Most preferably at least 80% of said cation-complexed anionic polysaccharide is insoluble in water.

Another feature of the present invention is that the water-insoluble, cation-complexed anionic polysaccharides are substantially free of underivatized cellulose. Preferably they comprise less than 20% underivatized cellulose by weight. More preferably they comprise less than 10% underivatized cellulose by weight. Still more preferably they comprise less than 5% underivatized cellulose by weight. Most preferably they comprise less than 1 % underivatized cellulose by weight.

Still another feature is that the water-insoluble, cation-complexed anionic polysaccharides are dry agglomerated particles that have a median size in the range of 4 mesh to 400 mesh or alternatively, 4750 microns to 38 microns. Preferably the median agglomerated particle size is between 1000 microns and 50 microns. More preferably the median agglomerated particle size is between 500 microns and 50 microns. Most preferably, the median agglomerated particle size is between 250 microns and 75 microns. The dry agglomerated particles, when added to an aqueous medium, readily disperse, and disaggregate to liberate smaller particles which readily disperse in the aqueous medium. The liberated smaller particles in the aqueous medium have a median particle size of less than 100 microns. Preferably, they have a median particle size of less than 50 microns. More preferably, they have a median particle size of less than 25 microns. Most preferably, they have a median particle size of less than 15 microns.

Some of the water-insoluble, cation-complexed anionic polysaccharides are fermentable. Fermentability of fibers is determined according to procedures well known to those skilled in the art. One such procedure can be found in European Patent Application publication 0,483,070,A2 published April 29, 1992 by Greenberg.

The preferred materials of the invention are readily dispersible into nutritional and health beverages, especially into nutritional beverages, such as an enteral formulation, using standard mixing techniques. The viscosity and physical appearance of the beverages are stable to high temperature treatments used to sterilize the product. They can be incorporated into an enteral formulation without significantly altering its viscosity, osmolality or stability. The aqueous media in which the cation-complexed anionic polysaccharides of the present invention can be employed include dairy drinks, such as milk, soy drinks such as soy milk, fruit juice and juice drinks such as orange and apple juice, enteral liquids such as ENSURE[®] manufactured by Ross Laboratories, division of Abbott Laboratories, infant formulas such as ENFAMIL[®] manufactured by Mead Johnson, and rehydration solutions. The materials of the present invention can also be blended into a dry mix which is reconstituted with water prior to being consumed. An ingestible liquid composition may contain from about 0.1 g to about 25 g of said polysaccharide per 100 ml of the aqueous medium. The upper limit is such that the viscosity of the nutritional liquid is less than 1000 cps measured at a shear rate of about 100 $sec^{-1}$. The lower limit is such that there is sufficient polysaccharide to cause an advantageous result. At the lower limit the ingestible composition comprises at least 0.1 gram of dispersed, water-insoluble, cation-complexed anionic polysaccharide per 100 ml of aqueous medium, preferably at least 0.3 g/100 ml and more preferably at least 0.5 g/100 ml. At the upper limit the ingestible composition comprises a maximum of 25 grams dispersed, water-insoluble, cation-complexed anionic polysaccharide per 100 ml of aqueous medium, preferably at most 10 g/100 ml and more preferably at most 5 g/100 ml. In addition, the compositions may be incorporated into many other types of foods including nutrient bars, cookies, bakery items, cereals, sauces, fruit spreads, meat spreads, seafood spreads, confectionery deserts, frozen deserts, refrigerated deserts, ice creams, yogurts, breads, soups, puddings, carbonated beverages, pureed or blenderized vegetables, meats and fruits, egg products, grain products, dairy items including flavored milks, butters and margarines, or pharmaceutical preparations including pills, powders or suspensions in combination with other efficacious and inert components including various drugs, clays, suspending agents, flavoring agents, buffering agents, coloring agents and sweetening agents. Solid foods may contain a concentration of water-insoluble, cation-complexed anionic polysaccharide of between 0.1 to 25 grams per serving.

The compositions of the present invention have an antidiarrheal efficacy in animals, particularly in mammals. Furthermore, these compositions have an antidiarrheal efficacy in humans. Examples of specific types of medically compromised mammals that would benefit from the therapeutic administration of the liquid nutritionals and other compositions of this invention includes humans having one or more of the following diseases, syndromes, anatomical conditions or symptoms: diabetes, cardiovascular disease, cancer, AIDS or HIV+, short bowel syndrome, ulcerative colitis, inflammatory bowel syndrome, Crohn's disease, renal disease, pulmonary disease, bacterial, viral or parasitic infections of the gastrointestinal tract, pancreatic disease, trauma, ulcers, diarrhea, constipation, fecal incontinence, loose stools, malabsorption, fistulas, ischemic bowel disease, hemorrhoids, sprue, Wipple's disease, antibiotic associated diarrhea, secretory diarrhea, osmotic diarrhea, lactose intolerance, radiation enteritis, ileostomy, diverticulitis, osteoporosis, mineral insufficiency, obesity or infantile colic.

The preferred water-insoluble, cation-complexed anionic polysaccharides are prepared by a novel high-solids/high-intensity wet milling process. The basic process consists mixing the polysaccharide, water and a source of cations with a high-intensity mixer. Preferably, the process comprises wetting out the polysaccharide with water to a total solids content of about 30 to 70%, preferably 40 to 60% and mixing in a high-intensity mixer in the presence of the cation reagent. Following mixing, the product may be dispersed "as is" in the aqueous medium, or dried, optionally ground and then redispersed in the aqueous medium; optionally, the product may be purified with water before direct utilization or drying; a further optional step is sterilization of the product prior to dispersion in a food or drug.

Prewetting the polysaccharide with water can be done in the high-intensity mixer itself, or in a separate lower-intensity mixer capable of distributive mixing high-solids formulations. The wetted mixture in this latter case would then be transferred to a high-intensity mixer. The choice is a matter of convenience and economics. Examples of lower-intensity mixers that are capable of distributive mixing, include, but are not limited to, tumbling blenders such as V-blenders, drum tumblers, double-cone blenders, and impeller blenders such as ribbon blenders, planetary mixers, food mixers/processors, conical-screw mixers, and sigma blade mixers. The water may optionally contain the complexing cation reagent(s) and, optionally, other conditioning agents such as acids or bases to control pH. In a preferred mode, however, the complexing cation reagent(s) is added as dry powder to the water-wetted polysaccharide in the high-intensity mixer.

The preferred high-intensity, or high-shear, mixers are capable of dispersive mixing by smearing and shearing action and preferably are capable of operating in the regime of 178.3 cal/sec (1 horsepower) to about 0.453-2.27 kg (2-4 lbs) of product. Calories/second per kilogram of material (horsepower per pound) is an industry term commonly used to describe the intensity conveyed by mixing equipment, and can be related to the work energy input to the material during mixing (See Plastics Compounding, 1992/1993 Redbook). Intensity of mixing increases as the cal/sec per kilogram (horsepower per pound) of material increases. Medium-intensity mixers operate in the regime of 178.3 cal/sec per 9.1 to 13.6 kg (20 to 30 lbs.) of material and low-intensity mixers operate at 178.3 cal/sec per 68-136 kg (150-300 lbs) of material. Low- to medium-intensity mixers, such as the tumbling or impeller blenders/mixers described above, may be adequate to produce materials suitable for texturized or thickened ingestible liquids or solid foods.

High-intensity mixers may be either batch or continuous. Examples of suitable batch high-intensity mixers are the Banbury made by the Ferrel Company, Ansonia, Connecticutt and similar design mixers such as made by Teledyne Specialty Equipment-Readco Products, York, Pennsylvania or Technical Machine Products, Cleveland, Ohio. Two- or multi-roll mills are also effective batch high-intensity mixers. Continuous units are commonly termed continuous Compounders or Processors. Preferred are the twin-screw, corotating, intermeshing screw or self-wiping blade type such as is manufactured by Teledyne Specialty Equipment-Readco Products, APV Chemical Machinery, Inc or Werner & Pfleiderer Corporation, Ramsey, New Jersey. Ferrel Company also makes a suitable continuous mixer/processor wherein the high-shear mixing section consists of rotors similar in cross-section to their Banbury batch mixer. A convenient laboratory high-intensity mixer that is designed to simulate the effect achieved in commercial equipment is the Brabender Prep Mixer coupled to a Brabender Plasti-Corder drive unit manufactured by C.W. Brabender Instruments, Inc., South Hackensack, New Jersey. Description and differentiation of mixing equipment with respect to intensive/dispersive and extensive/distributive can be found in (1) Plastics Compounding 1992/1993 Redbook pgs 112-133, (2) Perry's Chemical Engineers' Handbook, 6th edition, and (3) Polymer Processing by D.H. Morton-Jones, Chapman and Hall 1989, pgs 59-101.

Drying and grinding can be done on a wide variety of equipment -- the selection being based on the economic balance of investment and operating costs. Suitable dryers are described in Perry's Chemical Engineering Handbook, 6th edition, editied by Robert H. Perry, Don Green, and James Maloney, McGraw-Hill, 1984. These include direct contact batch and continuous dryers and indirect contact batch and continuous dryers. Preferred direct contact batch dryers are tray and fluid bed dryers. Preferred direct contact continuous dyers include pneumatic conveying dryers, continuous tray or belt dryers, and tunnel dryers. Preferred indirect batch dryers include agitated-pan dryers, vacuum-rotary dryers and vacuum-tray dryers. Preferred indirect continous dryers include drum dryers, screw-conveyor dryers, and vibrating-tray dryers. Suitable grinding equipment is also described in Perry's Chemical Engineering Handbook. These include jaw crushers, gyratory crushers, impact mills, shredders, rotary cutters, media mills, medium- and high-peripheral mills and fluid-energy mills. Pneumatic conveying dryers, such as the Aljet-Thermajet Flash Dryer manufactured by Fluid Energy Aljet, Plumsteadville, PA., are most preferred.

Convenient aspects of the process are (1) the formation of fine particle size materials without creating a dusting problem and (2) the formation of low-dusting, readily-dispersible, agglomerated granules upon drying. Low dusting is an important consideration in a commercial manufacturing operation. Polysaccharide polymer dusts are a nuisance and can form explosive dust-air mixtures. Dusting during the high-intensity mixing/milling operation is eliminated because the material is wetted with water and behaves as a cohesive mass. Attempts to achieve such small particle size materials by dry grinding would occasion a severe dusting problem. Dusting during drying is minimized because the small particles agglomerate to produce larger-size, lower-dusting particles. A desirable property of these dry agglomerates is that when introduced to aqueous media they readily dissaggregate and disperse to liberate smaller primary particles.

In animal studies the water-insoluble, cation-complexed anionic polysaccharides of the present invention have been found to improve the animal's ability to absorb water, their ability to absorb glucose, to reduce their weight loss, to solidify their feces and to increase intestinal cell proliferation.

As used herein, the term "nutritionally complete" refers to a feeding composition which contains carbohydrates, proteins, peptides and amino acids, essential fatty acids, vitamins and minerals in such amounts that a person can ingest only that composition for a prolonged period of time and obtain the recommended daily allowance of nutrients. The composition may have water added to it such that the composition is in liquid form and suitable for drinking or for use with a tube-feeding apparatus. Alternatively, the composition may be in dry form such as a powder or in other solid food forms such as a nutrient bar.

As used herein the term "ingestible" is meant to include all materials which are used by, or which perform a function in the body. Thus materials which are not adsorbed or absorbed are included as well as non-digestible and digestible materials.

As used herein the term "sterilization" means the use of a procedure, to reduce the microbial count of a foodstuff or pharmaceutical. The procedure used herein was steam autoclaving at 121°C for 20 minutes. The term "heat sterilizable" means that the use of heat does not cause a undesired increase to the viscosity of a liquid composition and does not result in significant coagulation or precipitation of the composition upon such heating.

Materials

HM Pectin: Genu Pectin BB Rapid Set from Hercules Incorporated, Wilmington Delaware; molecular weight is about 100,000 to 150,000 daltons; degree of methyl esterification is in the range of about 69%.

LM Pectin: Genu Pectin LM12CGZ from Hercules Incorporated, Wilmington Delaware; molecular weight is 60,000 to 100,000 daltons; degree of methyl esterification is 25-50%.

VLM Pectin HMW: Genu Pectin LM1912CSZ from Hercules Incorporated, Wilmington Delaware; molecular weight is 70,000 to 100,000 daltons; degree of methyl esterification about <5%; sodium or potassium salt of pectic acid.

VLM Pectin LMW: Obtained from Hercules Incorporated, Wilmington, Delaware, similar in composition to VLM Pectin HMW except molecular weight is 10,000 to 25,000 daltons.

LMA Pectin: Genu Pectin LM104ASZ from Hercules Incorporated, Wilmington Delaware; molecular weight is 100,000 to 150,000 daltons; degree of methyl esterification is 30-35%; degree of amidation is 15-25%

Sodium Alginate: from Aldrich Chemical Company, Milwaukee, Wisconsin # 18,094-7.

Carrageenan: Kappa-carrageenan from Sigma Chemical Co., St. Louis, Missouri # C-1263.

Gelatin A :from Sigma Chemical Co., #G-2625.

Metal Salts: from one or more of the following: Aldrich Chemical Co.; Pfaltz and Bauer, Inc., Waterbury, Connecticutt; J. T. Baker Inc., Philipsburg, New Jersey; Sigma Chemical Co.

Commercial Enterals: Ensure® and Osmolite® are products of Ross Laboratories, a division of Abbott Laboratories, Columbus, Ohio; Isocal® and Sustacal® are products of Mead Johnson Nutritionals, a Bristol-Myers Squibb Company, Evansville, Indiana; Enercal® Plus is a product of Wyeth Nutritionals, Inc., a Wyeth-Ayerst Company; Georgia, Vermont; Vivonex® T-E-N is a product of Clinical Products Division, Sandoz Nutrition Corporation, Minneapolis, Minnesota. The Table below lists the nutritional profiles for these enterals.

| ENTERAL FORMULATIONS' NUTRITIONAL PROFILE | | | | | | |
|---|---|---|---|---|---|---|
| | Ensure® 8 Fl. oz. (237 ml) | Osmolite® 8 Fl. oz., (237 ml) | Enercal® Plus per Liter | Sustacal® 8 Fl. oz. (237 ml) | Isocal® 8 Fl. oz. (237 ml) | Vivonex® T-E-N ® amount per 1000 ml standard dilution |
| Protein | 8.8 g | 8.8 g | 58 g | 14.5 g | 8.1 g | 38.2 g |
| Fat | 8.8 g | 9.1 g | 50.4 g | 5.5 g | 10.5 g | 2.77 g |
| Carbohydrate | 34.3 g | 34.3 g | 204 g | 33 g | 32 g | 205.57 g |
| Linoleic acid | | | | | | 2.17 g |
| Calories | 250 | 250 | 1500 | 240 | 250 | 1000 |
| Vitamin A | 625 IU | 625 IU | 1500 IU | 1110 IU | 630 IU | 2500 IU |
| Vitamin C | 37.5 mg | 37.5 mg | 200 mg | 13.3 mg | 38 mg | 60 mg |
| Vitamin $B_1$ | 0.38 mg | 0.38 mg | 1.5 mg | 0.33 mg | 0.48 mg | 1.5 mg |
| Vitamin $B_2$ | 0.43 mg | 0.43 mg | 1.8 mg | 0.4 mg | 0.54 mg | 1.7 mg |
| Niacin | 5 mg | 5 mg | 20 mg | 4.7 mg | 6.2 mg | 20 mg |
| Calcium | 125 mg | 125 mg | 1000 mg | 240 mg | 150 mg | 500 mg |
| Iron | 2.25 mg | 2.25 mg | 18 mg | 4 mg | 2.2 mg | 9.0 mg |
| Vitamin $B_6$ | 0.5 mg | 0.5 mg | 2.2 mg | 0.47 mg | 0.62 mg | 2.0 mg |
| Vitamin $B_{12}$ | 1.5 mcg | 1.5 mcg | 6 mcg | 1.33 mcg | 1.88 mcg | 6.0 mcg |
| Vitamin D | 50 IU | 50 IU | 400 IU | 89 IU | 50 IU | 200 IU |
| Vitamin E | 5.63 IU | 5.63 IU | 30 IU | 6.7 IU | 9.4 IU | 15 IU |
| Vitamin K | 10 mcg | 10 mcg | 100 mcg | 56 mcg | 31 mcg | 22.3 mcg |
| Folic Acid (Folacin) | 100 mcg | 100 mcg | 400 mcg | 89 mcg | 50 mcg | 0.4 mg |
| Biotin | 75 mcg | 75 mcg | 300 mcg | 67 mcg | 38 mcg | 0.3 mg |
| Choline | 75 mg | 75 mg | 400 mg | 56 mg | 62 mg | 73.7 mg |
| Pantothenic Acid | 2.5 mg | 2.5 mg | 10 mg | 2.3 mg | 3.1 mg | 10 mg |
| Sodium | 200 mg | 150 mg | 1100 mg | 220 mg | 125 mg | 460 mg |

(continued)

| ENTERAL FORMULATIONS' NUTRITIONAL PROFILE | | | | | | |
|---|---|---|---|---|---|---|
| | Ensure® 8 Fl. oz. (237 ml) | Osmolite® 8 Fl. oz. (237 ml) | Enercal® Plus per Liter | Sustacal® 8 Fl. oz. (237 ml) | Isocal® 8 Fl. oz. (237 ml) | Vivonex® T-E-N® amount per 1000 ml standard dilution |
| Potassium | 370 mg | 240 mg | 1875 mg | 490 mg | 310 mg | 782 mg |
| Chloride | 310 mg | 200 mg | 1700 mg | 350 mg | 250 mg | 819 mg |
| Phosphorus | 125 mg | 125 mg | 1000 mg | 220 mg | 125 mg | 500 mg |
| Magnesium | 50 mg | 50 mg | 400 mg | 90 mg | 50 mg | 200 mg |
| Manganese | 0.62 mg | 0.62 mg | 2.5 mg | 0.67 mg | 0.38 mg | 937 mcg |
| Iodine | 18.8 mcg | 18.8 mcg | 150 mcg | 33 mcg | 18.8 mcg | 75 mcg |
| Copper | 0.25 mg | 0.25 mg | 2.0 mg | 0.47 mg | 0.25 mg | 1 mg |
| Zinc | 2.82 mg | 2.82 mg | 15 mg | 3.3 mg | 2.5 mg | 10 mg |
| Se | 9 mcg | 9 mcg | | | 12.5 mcg | 50 mcg |
| Cr | 13 mcg | 13 mcg | | | 12.5 mcg | 16.7 mcg |
| Mo | 19 mcg | 19 mcg | | | 31 mcg | 50 mcg |
| L-carnitine | | 0.19 g | | | | |
| Taurine | | 0.19 g | | | | |
| mcg = micrograms IU = International Units mg = milligrams g = grams | | | | | | |

The following table shows the protein breakdown into amino acids of the 38.2 g of protein per serving of Vivonex®-T-E-N.

| Essential Amino Acids | Percent of 38.2 g Protein |
|---|---|
| L-Isoleucine | 8.27 |
| L-Leucine | 16.56 |
| L-Lysine | 5.10 |
| L-Methionine/Cystine | 3.66 |
| L-Phenylalanine | 5.16 |
| L-Threonine | 4.0 |
| L-Tryptophan | 1.28 |
| L-Valine | 8.27 |
| Total Essential Amino Acids | 52.3 |
| Non-essential Amino Acids | Percent of 38.2 g Protein |
| L-Alanine | 5.18 |
| L-Arginine | 7.64 |
| L-Aspartic Acid | 7.01 |
| L-Tyrosine | 0.84 |
| L-Glutamine | 12.85 |
| L-Histidine | 2.36 |
| Glycine | 4.01 |
| L-Proline | 4.88 |
| L-Serine | 2.93 |
| Total Non-essential Amino Acids | 47.7 |

General Procedures

Water Insolubles Measurement

Water insolubles were determined according to the following procedure: Cation-complexed anionic polysaccharides were dispersed in either deionized or distilled water at about a 10% wt/vol level. The mixture was stirred for 30 minutes at ambient temperature using a mechanical stirrer, and the solids isolated by centrifugation. This procedure was repeated three times. After the third wash, the product was dried to constant weight.

$$100 \times (\text{purified dried material weight/starting material weight}) = \% \text{ insolubles}.$$

Enterals Containing Cation-Complexed Anionic Polysaccharides

Cation-complexed materials were introduced into enterals at a 0.8-4% (wt/vol) loading by adding the appropriate amount of material to the commercial enteral formulation and homogenizing the mixture with a Tekmar Ultra-Turrax, Cincinnati, Ohio (model SD-45) homogenizer for three minutes at 60 percent of the maximum power level. The viscosity was measured at about 25°C on a Brookfield LVF viscometer equipped with a small sample adapter at speeds ranging from 6 to 60 RPM. This viscosity represented the before autoclaving viscosity. Samples having a viscosity less than 50 cps were measured at 60 RPM. Samples having a viscosity greater than 50 cps were measured at RPMs less than 60. Ten ml of sample was then placed in a 17 ml vial, loosely capped and autoclaved at 121°C for twenty minutes in a Vernitron Verna-Clave Sterilizer, Vernitron Medical Products, Inc. Carlstadt, New Jersey. After cooling to ambient temperature, the viscosity was again recorded; this value represented the after autoclaving viscosity.

Particle Size Determination in Water

Particle size measurements were performed in deionized water containing 0.25% w/w Tween 20 on a Horiba LA-900 laser scattering particle size distribution analyzer. Samples for analysis were prepared by dispersing approximately 100-500 mg in approximately 10-15 ml of 0.25% (wt./vol) aqueous Tween-20. The Tween-20 solution was filtered through a 0.20 μm nylon membrane filter prior to use.

Dry Particle Size Determination

The particle size distribution of the dry, ground product was determined by sieving a known quantity of material through US Standard Tyler Sieves of selected screen sizes on a sieve shaker. The quantity of material on each screen size was weighed and distribution determined by dividing the amount on each screen by the total sample weight.

Molecular Weight Determination of Pectins

The viscosity of a 0.1 % solution of pectin in a 1 % sodium hexametaphosphate solution was determined at 25°C by means of a Ubbelode viscometer. The viscosity of the solvent at 25°C was also determined. The ratio of the viscosity of the sample solution to the solvent is the relative viscosity. The molecular weight can be calculated from the relative viscosity.
A 0.05 gram sample of acid-washed pectin (see procedure for determining degree of esterification and galacturonic acid) is weighed into a 50 mL volumetric flask. Approximately 25 mL of 1% sodium hexametaphosphate solution is added and shaken overnight to dissolve the sample. The solution is diluted to the mark with additional hexametaphosphate solution and mix. The solution is equilibrated to 25°C in a water bath and then filtered into a Ubbelode viscometer. The flow time is recorded with a stopwatch. Similarly the flow time is recorded for the solvent alone.
Calculation:

$$(t - C/t) / (t_o - C/t_o) = \text{Relative Viscosity (RV)}$$

where:

C = kinetic energy correction constant for viscometer
t = flow time in seconds for the sample
$t_o$ = flow time in seconds for the solvent

$$6 (RV^{1/6} - 1) / (\% \text{ GA} \times 0.1 \times 4.75 \times 10^{-7}) = \text{Molecular Weight}$$

$$\text{Molecular Weight}/176 = \text{DP}$$

where:

RV = relative viscosity
GA = galacturonic acid (see procedure for determining degree of esterification and galacturonic acid)

Degree of Esterification and Galacturonic Acid Determinations of Pectins

The pectin sample is acid washed to an ash-free state, further washed until acid free, and then dried. A portion of the dried sample is dissolved and the free carboxylic acid titrated. The sample is then saponified by the addition of 0.5 N NaOH and stirred for 15 minutes. Finally an excess of 0.5 N HCl is added followed by titration of the excess acid. Values for the degree of esterification and galacturonic acid are calculated on the acid-washed dried basis.
A 2 to 3 gram sample of pectin is transferred to a 150 mL beaker with a stir bar. A 100 mL volume of 70/30 (v/v) isopropyl alcohol (IPA)-water mixture is added, followed by 5 mL concentrated HCl. The mixture is stirred rapidly for 10-15 minutes and filtered. The product is washed free of acid with 70/30 (v/v) IPA-water mixture. This procedure is followed by washing with isopropyl alcohol and drying the product. A 0.5 to 0.55 grams sample of dried acid-washed product is transferred to a 250 mL glass stoppered Erlenmeyer flask with a stir bar. Two mL of IPA are added to wet the sample, followed by 100 mL distilled water. The flask is stoppered and stirred until the entire sample is dissolved. Five drops of phenolphthalein indicator solution is added and the mixture is titrated with standardized 0.1 N NaOH. A measured volume of standardized 0.5 N NaOH (usually about 20 mL) is added to the neutralized sample, and stirred for 15 minutes. A measured volume of standardized 0.5 N HCL (usually about 18 mL) is slowly added and stirred vigorously until the gelatinous precipitate formed, redissolves and the solution becomes colorless. (If a pink color remains, additional 0.5 N HCl should be added). Back titration is performed with standardized 0.1 N NaOH to the first permanent pink color.

Calculation:

$$(mL_a \times N_a) \text{ / sample wt, g} = V_1$$

$$(mL_b \times N_b) - (mL_c \times N_c) + (mL_d \times N_a) \text{ / sample wt., g} = V_2$$

$$[V_2 \text{ / } (V_1 + V_2)] \times 100 = \text{Degree of Esterification}$$

$$(V_1 + V_2) \times 0.1941 \times 100 = \text{\% Galacturonic Acid}$$

where:

$mL_a$ = mL of 0.1 N NaOH consumed
$N_a$ = normality of standardized 0.1 N NaOH
$mL_b$ = mL of 0.5 N NaOH added
$N_b$ = normality of standardized 0.5 N NaOH
$mL_c$ = ml of 0.5 N HCl added
$N_c$ = normality of standardized 0.5 N HCl
$mL_d$ = ml of standardized 0.1 N NaOH used in the back titration

Enterals Containing Cation-Complexed Polysaccharides

Cation-complexed polysaccharides were introduced into commercial enterals at 0.8 to 4 % (wt/vol) loading by adding the appropriate amount of material to the commercial enteral formulation and homogenizing the mixture with a Tekmar Ultra-Turrax (model SD-45) homogenizer for 3 minutes at 60% of the maximum power level. The viscosity was measured on a Brookfield LVT viscometer; this value represented the viscosity before autoclaving (BA). Ten milliliters of sample was then placed in a 17 ml. vial, loosely capped, and autoclaved at 121°C for 20 minutes in a Vernitron Verna-Clave Sterilizer. After cooling to ambient temperature, the viscosity was again recorded; this value represented the after autoclaving (AA) viscosity.

Alternatively, a base formulation can be prepared, such as shown in the following Table.

| Base Formulation Table | |
|---|---|
| INGREDIENT | TOTAL ADDED PER 455 kg FINISHED PRODUCT |
| Canola Oil | 7.57 kg |
| High Oleic Safflower Oil | 4.62 kg |
| Medium Chain Triglycerides (Fractionated Coconut Oil) | 3.1 kg |
| Oil soluble Vitamin Lecithin | 680 g |
| Premix (containing Vitamin A, D, E and K)[1] | 27.3 g |
| Calcium Caseinate | 2.7 kg |
| Water | 346.7 kg |
| Ultra Trace Mineral/Trace Mineral Premix[2] | 109 g |
| Potassium Chloride | 0.385 kg |
| Potassium Iodide | 0.086 g |
| Magnesium Sulfate | 320 g |
| Magnesium Chloride | 840 g |
| Micronized Tricalcium Phosphate | 965 g |
| Hydrolyzed Corn Starch (Dextrose Equivalent 10.0) | 43.84 kg |
| Hydrolyzed Corn Starch (Dextrose Equivalent 20.0) | 14.6 kg |
| Sodium Caseinate | 17.64 kg |
| Potassium Citrate | 885 g |
| Sodium Citrate | 480 g |
| Cation-Complexed Polysaccharide | FIBER VARIED IN EXPERIMENTS |
| Ascorbic Acid | 242 grams |
| 45% Potassium Hydroxide | 126 grams |
| Choline Chloride | 252.5 g |
| Carnitine | 80.0 g |
| Water Soluble Vitamin Premix[3] | 75.2 g |
| Taurine | 70.2 g |

[1]Each gram of the premix provides about 106,400-115,400 IU Vitamin Palmitate,; 5,700-7,500 IU Vitamin D; 645-825 IU Vitamin E; 1,100-1,600 mg Vitamin K

[2]Each gram of the premix provides about: 77-88 mg zinc; 59-67 mg iron; 17-18 mg manganese; 7-8 mg copper; 2-3 mg selenium; 2-3 mg chromium; 5-6 mg molybdenum

[3]Each gram of the premix provides about 326-424 mg Niacinamide; 211-274 mg d-Calcium Pantothenate; 7-10 mg Folic Acid; 54-70 mg Thiamine Chloride Hydrochloride; 242-55 mg Riboflavin; 52-67 mg Pyridoxine Hydrochloride; 138-193 mg Cyanocobalarnin; 6-8 mg Biotin

During the blending together of the various slurries, described in the following text, the cation-complexed polysaccharides can be added to the blend to achieve the desired fiber concentration.

A protein-in-fat slurry is prepared by placing the canola oil, high oleic safflower oil and medium chain triglycerides oil to a tank and heating the oil blend to a temperature in the range of 140° to 150°F under agitation. The oil soluble vitamin lecithin is added to the oil blend, and then the vitamin premix is added to the oil blend. The calcium caseinate is added to the oil blend under agitation.

A carbohydrate/mineral slurry is prepared by placing about 56.2-59.4 kg (124 to 131 lbs) of water in a tank and heating the water to a temperature in the range of 145° to 160°F. The ultra trace mineral/trace mineral premix is added to the water and the mixture is agitated for five minutes. Add the potassium chloride potassium iodide, magnesium phosphate and tricalcium phosphate to the mixture with agitation. Add the hydrolyzed corn starch (dextrose equivalent 10.0) to the mixture and agitate thoroughly. Add the hydrolyzed corn starch (dextrose equivalent 20.0) to the mixture and mix well. Hold the mixture at a temperature in the range of 140° to 160°F.

A protein-in-water slurry is prepared by placing about 125.2 kg (276 lbs) of water in a tank and heating it to a temperature in the range of 145 to 155 °F Add the sodium caseinate to the water and agitate the mixture until the sodium caseinate is dissolved. Hold the slurry at a temperature in the range of 140° to 150°F.

Prepare a citrate slurry by placing about 124.7-127.9 kg (275-282 lbs) of water in a kettle and heating the water to a temperature in the range of 140° to 150°F Add the potassium citrate to the water with agitation. Add the sodium citrate to the mixture. Hold the slurry under agitation at a temperature in the range of 140° to 150°F.

Prepare a blend by first placing the citrate slurry in a blend tank and agitating it well, and then adding the carbohydrate/mineral slurry with agitation. The protein-in-water slurry is then added to the blend, the protein-in-fat slurry is then added to the blend. Cation complexed polysaccharide is added to the blend.

Following the blending step, the pH of each batch is adjusted to be in the range of 6.7-6.9 by adding a sufficient amount of potassium hydroxide to the blend.

Alternatively, the blend can be prepared from the ingredients set forth in the following Table, using the method described in the paragraphs immediately following the Table.

| INGREDIENT | TOTAL ADDED PER 455 kg FINISHED PRODUCT |
|---|---|
| Canola Oil | 4.72 kg |
| High Oleic Safflower Oil | 7.89 kg |
| Medium Chain Triglycerides (Fractionated Coconut Oil) | 3.17 kg |
| Oil soluble Vitamin Lecithin | 680 grams |
| Premix (containing Vitamin A, D, E and K)[1] | 27.2 grams |
| Calcium Caseinate | 2.75 kg |
| Water | 346.8 kg |
| Ultra Trace Mineral/Trace Mineral Premix[2] | 109 grams |
| Potassium Chloride | 385 grams |
| Potassium Iodide | 0.086 grams |
| Magnesium Phosphate | 952 grams |
| Micronized Tricalcium Phosphate | 966 grams |
| Hydrolyzed Corn Starch (Dextrose Equivalent 10.0) | 43.84 kg |
| Hydrolyzed Corn Starch (Dextrose Equivalent 20.0) | 14.6 kg |
| Sodium Caseinate | 17.64 kg |
| Potassium Citrate | 885 grams |
| Sodium Citrate | 480 grams |
| Cation Complexed Polysaccharide | VARIED IN EXPERIMENTS |
| Ascorbic Acid | 242.2 grams |
| 45% Potassium Hydroxide | 126 grams |
| Choline Chloride | 252.5 grams |
| Carnitine | 80.0 grams |
| Water Soluble Vitamin Premix[3] | 37.5 grams |
| Taurine | 70.2 grams |

[1]Each gram of the premix provides about 106,400-115,400 IU Vitamin Palmitate,; 5,700-7,500 IU Vitamin D; 645-825 IU Vitamin E; 1,100-1,600 mg Vitamin $K_1$

[2]Each gram of the premix provides about: 77-88 mg zinc; 59-67 mg iron; 17-18 mg manganese; 7-8 mg copper; 2-3 mg selenium; 2-3 mg chromium; 5-6 mg molybdenum

[3]Each gram of the premix provides about 326-424 mg Niacinamide; 211-274 mg d-Calcium Pantothenate; 7-10 mg Folic Acid; 54-70 mg Thiamine Chloride Hydrochloride; 242-55 mg Riboflavin; 52-67 mg Pyridoxine Hydrochloride; 138-193 mg Cyanocobalarnin; 6-8 mg Biotin

A protein-in-fat slurry is prepared by placing the canola oil, high oleic safflower oil and medium chain triglycerides oil in a tank and heating the oil blend to a temperature in the range of 140° to 150°F. under agitation. The oil soluble vitamin lecithin is added to the oil blend and then the vitamin premix is added to the oil blend. The calcium caseinate is added to the oil blend under agitation.

A carbohydrate/mineral slurry is prepared by placing about 56.2-59.4 kg (124 to 131 lbs) of water in a tank and heating the water to a temperature in the range of 145° to 160°F. The ultra trace mineral/trace mineral premix is added to the water and the mixture is agitated for five minutes. Add the potassium chloride, potassium iodide, magnesium phosphate and tricalcium phosphate to the mixture with agitation. Add the hydrolyzed corn starch (dextrose equivalent 10.0) to the mixture and agitate thoroughly. Add the hydrolyzed corn starch (dextrose equivalent 20.0) to the mixture and mix well. Hold the mixture at a temperature in the range of 140° to 160°F.

A protein-in-water slurry is prepared by placing about 125.2 kg (276 lbs.) of water in a tank and heating it to a temperature in the range of 145 to 155°F Add the sodium caseinate to the water and agitate the mixture until the sodium caseinate is dissolved. Hold the slurry at a temperature in the range of 140° to 150°F.

Prepare a citrate slurry by placing about 124.7-127.9 kg (275 to 282 pounds) of water in a kettle and heating the water to a temperature in the range of 140° to 150°F Add the potassium citrate to the water with agitation. Add the sodium citrate to the mixture. Hold the slurry under agitation at a temperature in the range of 140° to 150°F.

Prepare a blend by first placing the citrate slurry in a blend tank and agitating it well, and then adding the carbohydrate/mineral slurry with agitation. The protein in water slurry is then added to the blend, the protein-in-fat slurry is then added to the blend. Place all of the protein-in-fat slurry in a container and add the cation-complexed polysaccharide to it with agitation. Rinse the container with some of the blend to insure proper transfer. Add the protein-in-fat slurry to the blend, and rinse the container with some of the blend to insure proper transfer.

Use 1N potassium hydroxide to adjust the pH of the blend to be in the range of 6.7 to 6.9. Maintain the temperature of the blend in the range of 140° to 150°F. for a maximum of 2 hours before heat treatment and homogenization.

The blend is subjected to Ultra High Temperature Short Time (UHTST) heat treatment and homogenization by the following procedure. The blend is preheated to a temperature in the range of 155° to 165°F and then deaerated at 10 to 15 inches Hg. The blend is then emulsified at 900-1,000 psig. The blend is then heated to a temperature in the range of 229° to 231° F. and held at this temperature for a minimum of 10 seconds. The blend is then UHTST heat treated to a temperature of 292° to 294°F. with a minimum hold time of 5 seconds. If desired, the blend could instead be subjected to High Temperature Short Time heat treatment without adversely affecting product stability, as demonstrated in the preceding Table. The blend is then passed through a flash cooler to reduce the temperature of the bend to 248° to 252°F. The blend is then passed through a plate cooler to reduce the temperature of the blend to 160° to 170°F. The blend is then homogenized at 3,900-4,100/400-600 psig. The homogenized blend is held at a temperature of 165° to 175°F. for a minimum of 16 seconds. The blend is cooled to 34° to 44°F.

Prepare an ascorbic acid solution by adding to about 3.6 kg (8 lbs) of water the following ingredients: ascorbic acid, choline chloride, carnitine, 45% potassium hydroxide. Adjust the pH of this solution to be in the range of 6.0 to 10.0 using additional 45% potassium hydroxide. Add the ascorbic acid solution to the blend and mix thoroughly.

Prepare a vitamin/taurine solution by dissolving in about 2 kg (4.4 pounds) of water, the water soluble vitamin premix and taurine. Add this solution to the blend. Dilute the blend with the necessary amount of water to bring the percentage of total solid content, fat and protein to be within the desired ranges. Place the blend in suitable containers and then sterilize the product.

Example 1 - (in Table 1)

Calcium-complexed VLM Pectin was prepared. Thirty grams of VLM Pectin was mixed with 30 grams of water in a food processor. The wetted pectate was transferred to a Brabender 60 ml mixing/measuring head, fitted with roller blades and coupled to a Brabender Plasti-Corder drive unit. After about 15 minutes mixing, 1 gram calcium chloride dihydrate was slowly added. The mass was then mixed for about 60 minutes at 80-85°C. After cooling and discharging, the product was dried and ground. The sample analyzed for 69% water insolubility and had a median particle size diameter of 9 microns with distribution limits between about 1 to 68 microns when dispersed in water.

Examples 2-14 - (in Table 1)

A similar procedure to Example 1 was followed to make a series of calcium-complexed VLM Pectins varying in molecular weight, calcium content and purity. Table 1 lists the examples 2-14. The larger 125-150 gram charges of pectin were mixed in the Brabender Prep Mixer unit equipped with either roller or cam blades. Some of the examples included a purification step with water. This was done by mixing the calcium-complexed pectin with water at about 10% wt/wt in a container for about 30 minutes followed by centrifugation and decantation of the supernatant. This procedure was performed 3 times. After the last wash, the solids were collected and dried. These examples demonstrate that percent water insolubles greater than or equal to about 70% and median particle size diameters less than 15 microns (when dispersed in water) can be obtained at molar ratios of calcium to anhydrogalacturonic acid levels greater than or equal to about 0.1.

Example 14A

500 Grams of VLM Pectin was mixed with 500 grams of distilled water containing 20 grams of sodium hydroxide in a Henschel Mixer. The wetted mixture was then transferred to a 1.6 liter Banbury Mixer Manufactured by the Ferrel Company. After mixing for 5 minutes at 116 rpm, 170 grams of calcium propionate was added and mixing was continued for an additional 25 minutes. Ambient temperature water was circulated through the chamber jacket of the Banbury during the mixing operation. The product was then removed and batch washed in distilled water by slurrying in a centrifuge jar at about 10% solids, mixing for 20 minutes, followed by centrifugation and decantation of the supernatant. The washing procedure was repeated two more times. The purified wet cake was allowed to air dry in the hood overnight. This material was the dried at 70°C for twenty minutes in a Lab-Line Instruments, Inc. (Melrose Park, IL), High Speed Fluid Bed Drier, Model 23852. The dried material was then ground in a Retsch centrifugal grinding mill, Model ZM-1 (Retsch GmbH & Co. KG, Germany), through a 1 mm screen. The particle size distribution of the dried material was determined using 20, 60, 80, 200, and 325 mesh sieves (corresponding to 850, 250, 180, 75, and 45 microns). The weight percent on 20 mesh was 0.5%; on 60 mesh was 31.7%; on 80 mesh was 22.1%; on 200 mesh was 23.4%; on 325 mesh was 8.6%; and through 325 mesh was 13.7%.

The product showed a median particle size of 9.5 microns and a distribution ranging from 1.5 to 46.5 microns when dispersed in water. When dispersed in Ensure enteral at 2 weight percent loading, the viscosities before and after autoclaving were 17 cps and 16 cps, respectively.

Example 14B

400 Grams of VLM Pectin was mixed with 400 grams of distilled water containing 17.6 grams of sodium hydroxide in a Henschel Mixer. The wetted mixture was transferred to the Teledyne-Readco High Intensity Mixer. After mixing at 114 rpm for 1 minute, 86.3 grams calcium acetate was added in three portions over 3 minutes. Mixing was continued for an additional 20 minutes. The product was water washed, dried and ground as described in Example 14A.

The purified product showed a median particle size of 10.5 microns and a distribution of 3.0 to 59.0 microns when dispersed in water. When dispersed in Ensure enteral at 2% loading, the viscosities before and after autoclaving were 19 cps and 20 cps, respectively.

Example 14C

VLM Pectin and a 3 wt% sodium hydroxide solution were separately and continuously fed into the feed port of the Processor operating at 300 rpm with ambient temperature water circulating through the jacket surrounding the barrel. The feed rate of the VLM Pectin was about 4.5 kg/hr (10 lbs./hr), and the feed rate of the sodium hydroxide was about 6.8 kg/hr (15 lbs/hr). Calcium propionate was fed to the Processor through a second feed port about two-thirds down the length of the barrel at a rate of about 1.54 kg/hr (3.4 lbs/hr). The maximum temperature inside the barrel was controlled at about 75°C. After equilibration, the product was collected and purified by successive batch washing with water at about 10% total solids followed by centrifuging in a solid-bowl centrifuge. The purified wetcake was then dried in an Aljet Thermajet Flash drier, Model #3 (Fluid Energy Aljet, Plumsteadville, PA.). The particle distribution of the dried product was as follows: 0.7% on 20 mesh; 33.5% on 60 mesh; 19.6% on 80 mesh; 21.2% on 200 mesh; 9.4% on 325 mesh; and 15.6% through 325 mesh.

The purified product showed a median particle size of 8.9 microns and a distribution range of 1.5 to 77.3 microns when dispersed in water. When dispersed in Ensure enteral at 2% loading, the viscosities before and after autoclaving were 17 cps and 20 cps, respectively.

EXAMPLE 14D

453 Grams of VLM Pectin were blended with 453 grams of distilled water containing 18.1 grams of sodium hydroxide in a Teledyne-Readco CBM™ for five minutes. The wetted mixture was transferred to an Accurate feeder and fed at 4.5 kg/hr ( 10 lbs/hr) into the feed port of the continuous processor operating at 150 rpm. Ambient temperature water was circulating in the jacket surrounding the barrel. After one pass through the continuous processor, 907 grams of the product exiting the processor were reintroduced into the CBM™ and mixed with 154 grams of calcium propionate for 5 minutes. This mixture was then fed at a rate of 8.2 kg/hr (18 lbs/hr) into the feed port of the continuous processor operating at 150 rpm. The product was water washed, dried, and ground as described in Example 14A.

The purified product was dry blended with material of similar composition prepared in a Banbury Mixer according to Example 14A. The blended product showed a median particle size of 8.63 microns and a distribution of 1.7 to 68.0 microns dispersed in water. When dispersed in Ensure enteral at 2% loading, the viscosities before and after autoclaving were 19 cps and 25 cps, respectively. This blended product was designated polymer sample #15.

Examples 15-54 - (in Table 2)

A similar procedure to Example 1 was followed to make a series of cation-complexed polysaccharides varying in cation, type of polysaccharide and purity. Table 2 lists the Examples 15-54. The data in this table demonstrate the utility of cations in addition to calcium and polysaccharides in addition to pectins to produce cation-complexed polysaccharides.

Examples 55-70 (in Table 3)

Table 3 lists viscosity and osmolality data for various commercial enterals containing calcium-complexed VLM Pectins before and after sterilization by autoclaving.

The data in Table 3 demonstrate the minimal contribution of the calcium-complexed VLM Pectins to the viscosity and osmolality of several enteral formulations when incorporated at levels up to 4 grams/100 ml. Of special significance is the small change in viscosity data after sterilization by autoclaving. Examples 55-57 are comparative examples of pectins that are not complexed with cations. Uncomplexed, water-soluble HM-Pectins produce unacceptable high viscosity before sterilization by autoclaving, and coagulate the formulation during sterilization by autoclaving. Uncomplexed, water-soluble VLM Pectins produce desired viscosities before sterilization by autoclaving, but also induce coagulation of the formulation during sterilization by autoclaving.

Examples 71-75 - (in Table 4)

Table 4 lists viscosity data for various beverages containing calcium-complexed VLM Pectins before and after sterilization by autoclaving.

These data demonstrate that the calcium-complexed VLM Pectins of this invention remain insoluble and do not substantially contribute to the viscosity of the beverages even after sterilization by autoclaving. Uncomplexed, HM Pectins produce unacceptably high viscosities before sterilization by autoclaving and induce coagulation after sterilization by autoclaving. Uncomplexed VLM Pectins HMW or LMW form unstable dispersions after sterilization by autoclaving.

EXAMPLES 76-81 - (Table 5)

Examples 76 to 79 are comparative examples.

Example 76

Thirty four grams of a powdered nutritional (Enercal Plus; Wyeth Nutritionals, Inc.; see materials list for composition) was mixed with 76 milliliters water according to the manufacturer's specifications and homogenized until dissolved. The reconstituted product did not contain dietary fiber. The reconstituted product's viscosity was measured as in previous examples. Then the reconstituted product was examined for its utility as a diet for a subsequent animal tube-feeding study by pumping it through a 0.075 cm (0.030 inch) I.D. silastic gastrostomy tube at a flow rate of 2.5 milliliters per hour over a 24 hour period. Results appear in Table 5.

Examples 77-81A

In like manner, dietary fiber supplemented compositions were prepared by dry blending various pectins and calcium-complexed pectins with Enercal Plus or VIVONEX® T-E-N followed by reconstitution with water as described above (76 ml for Enercal Plus) or using 20.1 grams VIVONEX® T-E-N and 60.5 ml water. The dietary fiber concentration was 0.8% wt/vol in Enercal Plus and 2% wt/vol in the VIVONEX® T-E-N. Viscosity and utility as a diet for tube-feeding applications appear in Table 5.

The results show the utility of the present invention for reconstituted powdered nutritionals and for the reconstituted products in tube-feeding applications.

Examples 82-86 - (Table 6)

These examples illustrate the utility of the present invention for improving nutrient and water absorption by the gastrointestinal tract. The utility of the present invention as a means of nutritional therapy for mammals who have had a section of their bowel surgically removed is illustrated. Examples 82-86 are comparative examples.

Adult male Sprague Dawley rats weighing 300-350 gm were acclimated to the laboratory five days prior to surgery, during which time the animals were housed in individual, wire bottom metabolic cages, placed on a fiber-free pellet diet (Dyets Inc., Bethlehem, Pennsylvania), and allowed water ad libitum. Two days before the surgery, the rats were force fed 3 ml of castor oil by gavage needle in order to induce diarrhea and decrease the stool in the bowel at the time of surgery. The rats were fasted overnight with continued access to water on the evening prior to surgery.

On day six, all rats were weighed, anesthetized (Pentobarbital 50 mg/kg., i.p.), and underwent laparotomy and insertion of a 0.075 cm (0.030 inch) I.D. silastic gastrostomy tube for continuous enteral nutrition. The gastrostomy tube was tunneled out the back of the animal into a swivel-spring apparatus and attached to the interscapular muscles. Animals were then assigned to one of two operative groups. Control animals (transected) underwent transection and reanastomosis (interrupted 6-0 silk suture) of the small intestine 40 cm distal to the ligament of Treitz. The remaining animals (resected) underwent intestinal resection beginning 40 cm distal to the ligament of Treitz and including the entire small intestine distal to this point, the cecum, and the first centimeter of the proximal colon. Bowel continuity was restored by an end-to-side jejunocolic anastomosis of interrupted 6-0 silk suture one cm distal to the transected end of the colon, which was closed with a pursestring suture 6-0 silk. The 40 cm distance from the ligament of Treitz was measured using a 40 cm length of 3-0 silk suture placed along the mesenteric border of the gently stretched small intestine. All resected animals were therefore left with an equivalent length of proximal jejunum and no ileum. This resection reliably produces a syndrome which includes massive prolonged diarrhea, malabsorption of enteral nutrients, negative nitrogen balance, and body weight loss.

Postoperatively, the rats were randomly assigned to receive one of four continuous diet infusions via the gastrostomy tube. Continuous intragastric infusions were administered according to the following protocol: normal saline at 1.25 ml/hour during the evening following surgery; full-strength diet at 1.25 ml/hour on the first post-operative day (POD 1); and full-strength diet at 2.5 ml/hour on POD 2 through 8 (7 days). Diets infused at 2.5 ml/hour will provide 2.49 g N/kg body weight/day and 255 nonprotein kcals/kg body weight/day. The rats were offered water ad libitum throughout the study. Only qualitative features of stool consistency were noted due to unsuccessful attempts to quantitatively collect fecal matter.

On POD 9 anesthesia was administered as outlined previously. The rats were weighed, placed on a heating pad, and then under went laparotomy. The jejunal and colon segments were identified. Glucose absorption in the small bowel and water absorption in the large bowel were then measured by two simultaneous in vivo perfusion techniques.

A catheter (0,1625 cm, 0.065 in.) was placed in the proximal end of the jejunum, and a large catheter (0.3125 cm, 1/8 in. diameter) was placed in the distal end. The jejunum was placed back in the abdominal cavity, and the exteriorized ends of the catheter attached to a pump. A closed perfusion circuit was then created by pumping the glucose perfusate from a reservoir into the intestinal loop, and the efflux from the intestinal loop was fed back into the reservoir. Before beginning actual perfusion, the intestine was gingerly washed using warm, normal saline solution to clean it of residual contents, following which it was perfused at 2 ml/hour for three hours. The glucose perfusate contained [$^{14}$C] glucose (0.1 microCi/ml), 10 mm unlabeled glucose, [$^{3}$H] PEG (MW 4000, 0.5 microCi/ml), and sufficient Krebs buffer solution to make 15 ml of total perfusate. The pH was maintained at 7.40 by bubble stirring the perfusion solution in the mixing chamber with a mixture of 95% $O_2$ and $CO_2$. The PEG was used as a non-absorbable marker and functioned as an internal control for loss of substrate by processes other than absorption. During the perfusion, hourly 0.5 ml samples were taken from the reservoir for determination of labeled substances in a liquid scintillation counter, in order to obtain a time related glucose absorption curve expressed as micrograms (*ug*) glucose absorbed per cm intestine per hour.

Water absorption in the colon was measured concurrently. An inflow catheter (3M Tubing, size #15) was placed just distal to the jejunocolic anastomosis, and an outflow catheter was advanced through the anus and secured in the rectosigmoid with a silk ligature. Colonic contents were removed, the catheters exteriorized, and the colon returned to the abdominal cavity as described above. The rats were allowed to equilibrate for 30 minutes while the colonic segment was perfused with Krebs phosphate buffer at 0.6 ml/min. using the recirculation technique, in which a 25 ml reservoir of buffer at pH 6.8 and a [$^{3}$H] PEG (MW 4000) marker was used. During the 90 minute perfusion, 2 ml samples were drawn from the reservoir every 30 minutes for analysis by liquid scintillation counting. The resulting data are expressed as microliters (*ul*) water absorbed per cm colon per hour.

Following the absorption studies, the rats were euthanized by cardiac puncture and exsanguination. One-centimeter segments of jejunum and colon were obtained for histologic examination. The specimens were coded and immediately fixed in Bouin's solution. After H&E staining, the five tallest villi, their complete crypts, and the overall mucosal thickness were measured with an eye micrometer.

A one-way analysis of variance was used to test for intergroup differences. Tukey's HSD test was used to identify significant pairwise differences.

The enteral diet infusions used and the resulting data are shown in Table 6.

The average percent weight change for the group in Example 85 differs significantly from that of the group in Example 83 at a 90% confidence level. These results show that the present invention has utility for improving nutrient and water absorption.

Although the average water and glucose absorption for the groups in Examples 84-86 are not significantly different from that of the group in Example 83, at a 95% confidence level, the average water and glucose absorptions are consistently higher in the former example. These results show that the present invention has similar utility to water-soluble pectin for improving water and glucose absorption at the concentration used in this study.

EXAMPLES 87 - 89

The protocol described in the previous example(s) was repeated except for the following modifications to the protocol. Examples 87 and 88 are comparative examples. The water soluble HM pectin and the water insoluble calcium complexed VLM pectin were added to the powdered enteral diet at twice the concentration of the previous study. These powdered mixtures were then reconstituted with water to provide a final concentration of 1.6% weight/volume. Twenty one animals underwent resection. Postoperatively, the animals were randomlly assigned to one of three dietary groups giving N = 7 animals per dietary group. Stools were collected over postoperative days 6-8, pooled, and analyzed for percent water. Jejunal and colonic epithelial cell proliferation rates were measured by a standard technique. A transected control group was not included in this study.

The enteral diet infusions used and the resulting data are shown in Table 7.

At the higher levels of dietary fiber used in this study, several improvements were observed in those animals fed the calcium-complexed VLM pectin enriched diet relative to those animals fed the water soluble HM pectin enriched diet or the dietary fiber free diet. As shown in Table 7., these improvements included less body weight loss, less stool water, increased jejunal and colonic epithelial cell proliferation rates, and increased colonic water absorption. Furthermore, the animals fed the water insoluble calcium-complexed VLM pectin enriched diet had, qualitatively, the firmest stools.

Thus, at the higher levels of dietary fiber used in this study, it is apparent that the water insoluble, cation-complexed anionic polysaccharide of this invention is useful as a means for maintaining body weight, decreasing diarrhea, improving nutrient absorption and increasing intestinal epithelial cell proliferation rates. Furthermore, it is apparent that the water insoluble, cation complexed anionic polysaccharide of this invention is an improvement over the prior art water soluble pectin and that it is preferred as a dietary fiber for the nutritional management of mammals having a underlying disease or condition accompanied by diarrhea or nutrient malabsorption.

In summary, the results indicate that the present invention has utility for providing nutritional therapy for mammals suffering from short bowel syndrome. The improved stool consistency suggests that the present invention has utility for decreasing diarrheal disturbances and that it has utility for decreasing stomal effluent output.

EP 0 648 495 B1

TABLE 1

CALCIUM-COMPLEXED PECTATES PREPARED FROM VLM PECTIN AND CALCIUM CHLORIDE IN A BRABENDER HIGH-INTENSITY MIXER

| Example # | Polymer # | VLM Pectin Type | [VLM Pectin] (g) | [H₂O] (g) | [CaCl₂·H₂O] (g) | Ca/AGA Molar Ratio (a) | Purified | Dried | % Water Insolubles | Particle Size Median Distribution (microns) | (microns) |
|---|---|---|---|---|---|---|---|---|---|---|---|
| 1 | 1 | HMW | 30 | 30 | 1 | 0.055 | No | Yes | 69 | 9 | 1-68 |
| 2 | 2 | HMW | 30 | 30 | 2 | 0.11 | No | Yes | 75 | 10 | 2-77 |
| 3 | 3 | HMW | 30 | 30 | 3 | 0.17 | No | Yes | 80 | 9 | 2-57 |
| 4 | 4 | HMW | 30 | 30 | 4 | 0.22 | No | Yes | 78 | 8 | 2-52 |
| 5 | 5 | HMW | 30 | 30 | 4 | 0.22 | No | Yes | 79 | 7 | 1-30 |
| 6 | 6 | HMW | 150 | 150 | 16.5 | 0.18 | No | Yes | 87 | 10 | 2-89 |
| 7 | 7 | ----SAME AS EXAMPLE 6 EXCEPT PURIFIED---- | | | | | Yes | Yes | nd | nd | nd |
| 8 | 8 | HMW | 150 | 150 | 18.5 | 0.2 | No | Yes | 86 | 10 | 62-89 |
| 9 | 9 | ----SAME AS EXAMPLE 8 EXCEPT PURIFIED---- | | | | | Yes | Yes | 86 | nd | nd |
| 10 | 10 | HMW | 125 | 125 | 16.7 | 0.22 | Yes | Yes | nd | 11 | 2-90 |
| 11 | 11 | HMW | 150 | 150 | 30 | 0.33 | No | Yes | 82 | 7 | 1-45 |
| 12 | 12 | LMW | 150 | 150 | 30 | 0.33 | No | Yes | nd | 9 | 1-101 |
| 13 | 13 | LMW | 125 | 125 | 16.7 | 0.22 | Yes | Yes | nd | nd | nd |
| 14 | 14 | LMW | 30 | 30 | 4 | 0.22 | No | Yes | 61 | nd | nd |

(a) Molar ratio of the calcium ion added to the anhydrogalacturonic acid content in the pectin.

TABLE 2
OTHER CATION-COMPLEXED POLYSACCHARIDE COMPOSITIONS PREPARED IN A BRABENDER HIGH-INTENSITY MIXER

| Example # | Polysaccharide | Cation Reagent | [Cation Reagent] (grams) | [Polysaccharide] (grams) | [H₂O] (grams) | % Water Insolubles | Particle Size (Dispersed In Water) Median (microns) | Distribution (microns) | 2% Viscosity in ENSURE, cps Unpurified Sample Before After Autoclaving | After Autoclaving | Purified Sample Before Autoclaving | |
|---|---|---|---|---|---|---|---|---|---|---|---|---|
| 15 | VLM Pectin | Calcium chloride dihydrate | 2.4 | 27.2 | 25 | 84 | 6 | 1-26 | 22 | >1000 | 14 | 21 |
| 16 | VLM Pectin | Calcium citrate tetrahydrate | 13.5 | 27.2 | 25 | 69 | 10 | 3-45 | 10 | 9 | 12 | 26 |
| 17 | VLM Pectin | Calcium citrate tetrahydrate | 5 | 30 | 30 | 81 | nd | nd | 13 | 15 | nd | nd |
| 18 | VLM Pectin | calcium sulfate dihydrate | 4 | 27.2 | 25 | 73 | 8 | 3-77 | 13 | 141 | 18 | 17 |
| 19 | VLM Pectin | calcium phosphate | 6 | 27.2 | 25 | 75 | 7 | 1-39 | 17 | 207 | 22 | 19 |
| 20 | VLM Pectin | Calcium Ascorbate | 10.1 | 27.2 | 25 | 64 | 5 | 1-20 | 16 | 41 | 10 | 12 |
| 21 | VLM Pectin | Calcium Hydroxide | 1.8 | 27.2 | 25 | 77 | 5 | 1-17 | nd | nd | 11 | 12 |
| 22 | VLM Pectin | Calcium Propionate | 21 | 125 | 125 | 80 | 11 | 2-88 | nd | nd | 10 | 12 |
| 23 | VLM Pectin | Calcium Acetate Monohydrate | 17.9 | 125 | 125 | 84 | 10 | 2-77 | nd | nd | 12 | 144 |
| 24 | VLM Pectin | Calcium Gluconate | 38.9 | 100 | 100 | 68 | 11 | 2-101 | nd | nd | 15 | 23 |
| 25 | VLM Pectin | Ferrous Chloride | 10.9 | 125 | 125 | 62 | 8 | 1-51 | nd | nd | 130 | coagulated |
| 26 | VLM Pectin | Ferric Citrate | 6 | 125 | 125 | 41 | 16 | 2-101 | nd | nd | 231 | coagulated |
| 27 | VLM Pectin | Zinc Chloride | 6.5 | 39 | 35 | nd | 6 | 2-23 | nd | nd | 11 | coagulated |
| 28 | VLM Pectin | Magnesium Sulfate Calcium Propionate | 3 9.3 | 27.5 | 37.5 | nd | 7 | 1-45 | nd | nd | 20 | 19 |
| 29 | VLM Pectin | Magnesium Sulfate Calcium Propionate | 6 7.5 | 27.5 | 37.5 | nd | 7 | 1-51 | nd | nd | 20 | 19 |

EP 0 648 495 B1

| Example # | Polysaccharide | Cation Reagent | [Cation Reagent] (grams) | [Polysaccharide] (grams) | [H₂O] (grams) | % Water Insolubles | Particle Size (Dispersed In Water) Median (microns) | Distribution (microns) | 2% Viscosity in ENSURE, cps Unpurified Sample Before After Autoclaving | After Autoclaving | Purified Sample Before Autoclaving | |
|---|---|---|---|---|---|---|---|---|---|---|---|---|
| 30 | VLM Pectin | Calcium Chloride Dihydrate Ferrous Sulfate | 4 8.7 | 33 | 30 | nd | 57 | 2-395 | nd | nd | 192 | nd |
| 31 | VLM Pectin | Albumin Magnesium Sulfate Calcium Propionate | 20 2.1 9.3 | 20.5 | 25 | 70 | 30 | 1-394 | nd | nd | 11 | 19 |
| 32 | VLM Pectin | Albumin Magnesium Sulfate Calcium Propionate | 16.7 3.7 4.6 | 17 | 21 | 69 | 35 | 1-344 | nd | nd | 15 | nd |
| 33 | VLM Pectin | Casein Calcium Propionate Magnesium Sulfate | 12.5 4.5 3.6 | 12.5 | 25 | nd | 8 | 1-45 | nd | nd | 19 | 22 |
| 34 | VLM Pectin | Gelatin A Magnesium Sulfate Calcium Propionate | 16.7 1.7 4.5 | 16.5 | 25 | 83 | 145 | 3-777 | nd | nd | 18 | 19 |
| 35 | VLM Pectin (Potassium Salt) | Calcium Chloride Dihydrate | 16.7 | 125 | 125 | 76 | 13 | 2-133 | nd | nd | 17 | 19 |
| 36 | Pectic Acid | Calcium Hydroxide | 6.8 | 125 | 125 | 73 | 8 | 1-59 | nd | nd | 102 | 27 |
| 37 | Pectic Acid | Calcium Hydroxide | 3.7 | 35 | 35 | nd | nd | nd | nd | nd | 12 | 22 |
| 38 | LM Pectin | Ferric Citrate | 6 | 125 | 125 | 43 | 5 | 1-30 | nd | nd | 246 | coagulated |
| 39 | LM Pectin | Calcium Chloride Dihydrate | 6 | 30 | 30 | 43 | 24 | 4-175 | 31 | coagulated | nd | 23 |

EP 0 648 495 B1

| Example # | Polysaccharide | Cation Reagent | [Cation Reagent] (grams) | [Polysaccharide] (grams) | [H₂O] (grams) | % Water Insolubles | Particle Size (Dispersed in Water) Median (microns) | Distribution (microns) | 2% Viscosity in ENSURE, cps — Unpurified Sample Before Autoclaving | After Autoclaving | Purified Sample Before Autoclaving | After Autoclaving |
|---|---|---|---|---|---|---|---|---|---|---|---|---|
| 40 | LM Pectin | Calcium Chloride Dihydrate | 3 | 30 | 30 | 43 | nd | nd | 9 | coagulated | nd | nd |
| 41 | LM Pectin | Calcium Hydroxide | 6 | 30 | 30 | 62 | nd | nd | 7 | 9 | nd | nd |
| 42 | LM Pectin | None | — | — | — | — | — | — | >1000 | coagulated | nd | nd |
| 43 | LMA Pectin | Calcium Chloride Dihydrate | 3 | 30 | 30 | 74 | 9 | 2-68 | >1000 | coagulated | nd | nd |
| 44 | LMA Pectin | Calcium Chloride Dihydrate | 6 | 30 | 30 | 75 | 11 | 2-89 | 27 | coagulated | nd | nd |
| 45 | HM Pectin | None | 0 | — | — | — | — | — | — | — | >1000 | coagulated |
| 46 | HM Pectin | Calcium Chloride Dihydrate | 4.5 | 30 | 30 | 0 | nd | nd | nd | nd | nd | nd |
| 47 | HM Pectin | Calcium Hydroxide | 3 | 30 | 30 | 66 | nd | nd | nd | nd | 16 | 58 |
| 48 | HM Pectin | Calcium Hydroxide | 2.5 | 30 | 30 | 79 | nd | nd | nd | nd | 10 | 31 |
| 49 | Sodium Alginate | None | 0 | — | — | — | — | — | — | — | coagulated | nd |
| 50 | Sodium Alginate | Calcium Chloride Dihydrate | 14 | 150 | 300 | <1 | nd | nd | nd | nd | nd | nd |
| 51 | Sodium Alginate | Calcium Chloride Dihydrate | 20 | 150 | 150 | nd | nd | nd | nd | nd | 11 | 59 |
| 52 | K-Carrageenan | None | 0 | — | — | — | — | — | >1000 | coagulated | nd | nd |
| 53 | K-Carrageenan | Calcium Chloride Dihydrate | 6 | 30 | 30 | nd | 16 | 1-40 | nd | nd | 32 | coagulated |

25

| Example # | Polysaccharide | Cation Reagent | [Cation Reagent] (grams) | [Polysaccharide] (grams) | [H₂O] (grams) | % Water Insolubles | Particle Size (Dispersed In Water) | | 2% Viscosity in ENSURE, cps | | | |
|---|---|---|---|---|---|---|---|---|---|---|---|---|
| | | | | | | | Median (microns) | Distribution (microns) | Unpurified Sample | | Purified Sample | |
| | | | | | | | | | Before Autoclaving | After Autoclaving | Before Autoclaving | After Autoclaving |
| 54 | K-Carrageenan | Ferrous Chloride Tetrahydrate | 5.9 | 35 | 35 | nd | 200 | 2-900 | nd | nd | 305 | nd |

Examples 34, 42, 45, 49, 52 and 54 are comparative examples.

## TABLE 3
## VISCOSITY DATA OF CALCIUM-COMPLEXED PECTATES IN VARIOUS COMMERCIAL ENTERALS

| Example # | Polymer # (From Table 1) | [Polymer] (wt/vol%) | ENSURE VISCOSITY, cps | | | OSMOLITE VISCOSITY, cps | | | ISOCAL VISCOSITY, cps | | | SUSTACAL VISCOSITY, cps | |
|---|---|---|---|---|---|---|---|---|---|---|---|---|---|
| | | | Osmolality mosmoles/kg | Before Autoclaving | After Autoclaving | Osmolality mosmoles/kg | Before Autoclaving | After Autoclaving | Osmolality mosmoles/kg | Before Autoclaving | After Autoclaving | Before Autoclaving | After Autoclaving |
| 55 | HM PECTIN | 2 | nd | >1000 | Coagulated | nd | nd | nd | nd | nd | nd | nd | nd |
| 56 | VLM PECTIN HMW | 2 | nd | 21 | Coagulated | nd | nd | nd | nd | nd | nd | nd | nd |
| 57 | VLM PECTIN LMW | 2 | nd | 15 | Coagulated | nd | 13 | Coagulated | nd | 22 | Coagulated | 24 | Coagulated |
| 58 | 10 | 2 | 421 | 14 | 36 | 271 | 12 | 22 | nd | nd | nd | nd | nd |
| 59 | 3 | 2 | nd | ND | nd | nd | nd | nd | nd | nd | nd | 24 | 196 |
| 60 | 6 | 2 | nd | 22 | 118 | nd | 14 | 24 | nd | 16 | 35 | nd | nd |
| 61 | 7 | 2 | nd | 15 | 29 | nd | 12 | 16 | nd | 20 | 18 | nd | nd |
| 62 | 9 | 2 | nd | 14 | 26 | nd | 12 | 19 | nd | 17 | 20 | nd | nd |
| 63 | 8 | 2 | nd | 15 | 57 | nd | 16 | 19 | nd | 17 | 33 | 17 | 164 |
| 64 | 5 | 2 | nd | ND | nd | nd | 22 | 16 | nd | nd | nd | nd | nd |
| 65 | NONE | 0 | 412 | 12 | 11 | 256 | 14 | 12 | 231 | 14 | 11 | nd | nd |
| 66 | 10 | 1 | 418 | 11 | 12 | 266 | 14 | 13 | 238 | 14 | 12 | nd | nd |
| 67 | 10 | 2 | 427 | 17 | 22 | 265 | 17 | 20 | 238 | 20 | 18 | nd | nd |
| 68 | 10 | 3 | 430 | 19 | 47 | 264 | 22 | 40 | 253 | 24 | 25 | nd | nd |
| 69 | 10 | 4 | 420 | 30 | 101 | 271 | 56 | 106 | 267 | 57 | 30 | nd | nd |
| 70 | 13 | 2 | | 26 | 36 | | 21 | 34 | nd | 27 | 30 | nd | nd |

Examples 55-57 and 65 are comparative examples.

EP 0 648 495 B1

## TABLE 4
### VISCOSITY OF CALCIUM-COMPLEXED PECTATES IN VARIOUS BEVERAGES AT 2 WEIGHT/VOLUME PERCENT LOADING

| EXAMPLE # | POLYMER# | GATORADE VISCOSITY, cps | | WHOLE MILK VISCOSITY, cps | | ENFAMIL INFANT MILK VISCOSITY, cps | | SKIM MILK VISOCSITY, cps | |
|---|---|---|---|---|---|---|---|---|---|
| | | Before Autoclaving | After Autoclaving | Before Autoclaving | After Autoclaving | Before Autoclaving | After Autoclaving | Before Autoclaving | After Autoclaving |
| 71 | HM PECTIN | nd | nd | 480 | Coagulated | 440 | Coagulated | nd | nd |
| 72 | VLM PECTIN LMW | nd | nd | 6 | 24 (Unstable Dispersion) | 6 | 6 (Unstable Dispersion) | nd | nd |
| 73 | VLM PECTIN LMW | nd | nd | 3 | 3 | 43 | Coagulated | nd | nd |
| 74 | 10 | 3 | 3 | 4 | 4 | 4 | 4 | 6 | 6 |
| 76 | 13 | 3 | 4 | nd | nd | nd | nd | nd | nd |

Examples 71-73 are comparative examples.

TABLE 5

| RECONSTITUTABLE POWDERED NUTRITIONALS AND UTILITY FOR TUBE-FEEDING APPLICATIONS | | | | |
|---|---|---|---|---|
| EXAMPLE # | POLYMER # | VISCOSITY (cps) | TUBE-FEEDING STABILITY | COMMENTS |
| 76 | NONE | 11 | GOOD | UNIMPEDED FLOW THROUGH TUBE OVERNIGHT |
| 77 | HM PECTIN | 132 | GOOD | UNIMPEDED FLOW THROUGH TUBE OVERNIGHT |
| 78 | VLM PECTIN HMW | 27 | POOR | FLOW THROUGH TUBE STOPPED BECAUSE OF CLOGGING |
| 79 | VLM PECTIN LMW | 13 | POOR | FLOW THROUGH TUBE STOPPED BECAUSE OF CLOGGING |
| 80 | 10 | 11 | GOOD | UNIMPEDED FLOW THROUGH TUBE OVERNIGHT |
| 81 | 13 | 12 | GOOD | UNIMPEDED FLOW THROUGH TUBE OVERNIGHT |
| 81A | 15 | 19 | ND | |
| Examples 76-81 ENERCAL PLUS - Examples 76-79 are comparative examples. Example 81A VIVONEX T-E-N | | | | |

## TABLE 6
### SHORT BOWEL SYNDROME ENTERAL NUTRITION STUDY

| EXAMPLE # | POLYMER # | SURGERY GROUP | STOOL CONSISTENCY | WEIGHT CHANGE | | GLUCOSE ABSORPTION | | WATER ABSORPTION | |
|---|---|---|---|---|---|---|---|---|---|
| | | | | VALUE (%) | STANDARD DEVIATION | VALUE (ug/cm/hr) | STANDARD DEVIATION | VALUE (ul/cm/hr) | STANDARD DEVIATION |
| 82 | NONE | TRANSECTED | PELLETS | +2.5 | 2.37 | 1.9 | 0.9777 | 285 | 34 |
| 83 | NONE | RESECTED | UNFORMED, WATERY | -8.6 | 3.87 | 2.4 | 0.4843 | 351 | 40 |
| 84 | HM PECTIN | RESECTED | FORMED | -7.4 | 4.66 | 3.1 | 0.9924 | 380 | 49 |
| 85 | 10 | RESECTED | FORMED | -4.0 | 2.46 | 2.8 | 0.2531 | 388 | 17 |
| 86 | 13 | RESECTED | FORMED | -7.1 | 1.61 | 3.0 | 0.421 | 384 | 34 |

### Table 7.

### SHORT BOWEL SYNDROME ENTERAL NUTRITION STUDY

### COMPARISON OF WATER SOLUBLE HM PECTIN AND WATER INSOLUBLE CALCIUM COMPLEXED VLM PECTIN

### AT A CONCENTRATION OF 1.6% WEIGHT/VOLUME

| EXAMPLE # | POLYMER # | SURGERY GROUP | STOOL CONSISTENCY | BODY WEIGHT CHANGE IN GRAMS | PERCENT WATER IN STOOL, % | JEJUNAL EPITHELIAL CELL PROLIFERATION RATE$^C$, % | COLONIC EPITHELIAL CELL PROLIFERATION RATE$^C$, % | COLONIC WATER ABSORPTION (microL/cm/hr) | JEJUNAL GLUCOSE ABSORPTION (micromol/cm²/hr) |
|---|---|---|---|---|---|---|---|---|---|
| 87 | NONE | RESECTED | UNFORMED, WATERY | -49 +/- 5 | 90 +/- 1.3 | 29 +/- 3 | 16 +/- 2 | 289 +/- 14 | 1.2 +/- 0.1 |
| 88 | HM PECTIN | RESECTED | FORMED, SOFT | -49 +/- 6 | 95 +/- 1.1$^A$ | 38 +/- 3$^B$ | 21 +/- 2 | 322 +/- 16$^B$ | 1.9 +/- 0.2 |
| 89 | 15 | RESECTED | FORMED, FIRMEST | -19 +/- 8$^A$ | 93 +/- 1.1$^B$ | 51 +/- 3$^A$ | 26 +/- 2$^A$ | 358 +/- 16$^B$ | 1.5 +/- 0.2 |

Data are mean +/- standard error mean ; superscript A = p<0.05 vs. Example 87 and Example 88 ; superscript B = p<0.05 vs. Example 87 superscript C = expressed as percentage of epithelial cells mitotically active by bromodeoxyuridine labelling.

**Claims**

1. A substantially water-insoluble, cation-complexed anionic polysaccharide, said cation being selected from alkaline earth metal cations, alkali metal cations, transition metal cations, protein, amino acids, oligopeptides, cationic or amino-functionalized polysaccharides, cationic or amino-functionalized synthetic polymers, or combinations thereof, wherein said cation-complexed anionic polysaccharide comprises particles in a dry, agglomerated form that readily disperse and disaggregate when added to an aqueous medium to liberate smaller particles having a median particle size less than 100 $\mu$m.

2. A substantially water-insoluble, cation-complexed anionic polysaccharide according to Claim 1, wherein said cation is selected from calcium, iron, magnesium, zinc, potassium, sodium, copper and manganese, or mixtures thereof.

3. A substantially water-insoluble, cation-complexed anionic polysaccharide according to Claim 1 or Claim 2 wherein said anionic polysaccharide is selected from carboxylated polysaccharides, sulfated polysaccharides, and phosphated polysaccharides.

4. A substantially water insoluble, cation-complexed anionic polysaccharide according to Claim 3 wherein the carboxylated polysaccharides are selected from alginates, pectins, arabic, karaya, tragacanth, ghatti, psyllium, flax seed, okra, xanthan, gellan, carboxymethyl cellulose, carboxymethyl guar, carboxymethyl starch and hyaluronic acid; the sulfated polysaccharides are selected from carrageenan, heparin, chondroitin sulfate, dermatan sulfate, heparan sulfate, keratan sulfate, keratan and cellulose sulfate; and the phosphated polysaccharides are selected from phosphated cellulose, phosphated starch and phosphated glucan.

5. An ingestible composition comprising the substantially water-insoluble, cation-complexed anionic polysaccharides as defined in any preceding Claim dispersed in an aqueous medium wherein said ingestible composition has a low viscosity and is heat sterilizable.

6. An ingestible composition according to Claim 5 further comprising at least one composition selected from amino acids, peptides and proteins.

7. Use of a substantially water-insoluble, cation-complexed anionic polysaccharide according to any of Claims 1 to 4 for preparing an ingestible composition for treating weight loss in an animal, wherein a therapeutically effective amount of said ingestible composition is enterally administered to said animal.

8. Use of a substantially water insoluble, cation-complexed anionic polysaccharide according to any of Claims 1 to 4 for preparing an ingestible composition for treating diarrhea in an animal, wherein a therapeutically effective amount of said ingestible composition is enterally administered to said animal.

9. Use according to Claim 7 or Claim 8, wherein the animal has had a part of its gastrointestinal tract removed by a surgical procedure.

10. A method of preparing a substantially water-insoluble, heat sterilizable, cation-complexed anionic polysaccharide as defined in any of Claims 1 to 4 comprising wetting a substantially water-soluble anionic polysaccharide with water to a total solids level of about 30 to 70%, adding a source of cations followed by mixing in a high intensity mixer to a total solids content of about 30 to 70%.

11. A low viscosity, heat sterilizable feeding composition comprising: carbohydrates providing approximately 1 to 99% of total calories; protein, peptides and amino acids providing approximately 1 to 99% of total calories; lipid containing essential fatty acids providing approximately 1 to 99% of total calories; vitamins; minerals; water; and at least one substantially water-insoluble, cation-complexed anionic polysaccharide as defined in any of Claims 1 to 4.

12. A composition comprising: carbohydrates providing approximately 1 to 99% of total calories; protein, peptides and amino acids providing approximately 1 to 99% of total calories; lipid containing essential fatty acids providing approximately 1 to 99% of total calories; vitamins; minerals; and at least one substantially water-insoluble, cation-complexed anionic polysaccharide as defined in any of Claims 1 to 4.

13. A food comprising a composition as defined in Claim 5 or Claim 6.

**14.** A pharmaceutical comprising a composition as defined in Claim 5 or Claim 6.

**Patentansprüche**

**1.** Im wesentlichen wasserunlösliches, mit Kationen komplexiertes, anionisches Polysaccharid, wobei das Kation ausgewählt ist Erdalkalimetallkationen, Alkalimetallkationen, Übergangsmetallkationen, Proteinen, Aminosäuren, Oligopeptiden, kationischen oder Amino-funktionalisierten Polysacchariden, kationischen oder Amino-funktionalisierten synthetischen Polymeren oder Kombinationen daraus, worin das mit Kationen komplexierte, anionische Polysaccharid Teilchen in trockener, agglomerierter Form umfaßt, die leicht dispergieren und disaggregieren, wenn sie in ein wäßriges Medium gegeben werden, wobei sie kleinere Teilchen mit einer mittleren Teilchengröße von weniger als 100 µm freisetzen.

**2.** Im wesentlichen wasserunlösliches, mit Kationen komplexiertes, anionisches Polysaccharid gemäß Anspruch 1, worin das Kation ausgewählt ist aus Calcium, Eisen, Magnesium, Zink, Kalium, Natrium, Kupfer und Mangan oder Mischungen daraus.

**3.** Im wesentlichen wasserunlösliches, mit Kationen komplexiertes, anionisches Polysaccharid gemäß Anspruch 1 oder 2, worin das anionische Polysaccharid ausgewählt ist aus carboxylierten Polysacchariden, sulfatierten Polysacchariden und phosphatierten Polysacchariden.

**4.** Im wesentlichen wasserunlösliches, mit Kationen komplexiertes, anionisches Polysaccharid gemäß Anspruch 3, worin die carboxylierten Polysaccharide ausgewählt sind aus Alginaten, Pektinen, Gummi arabicum, Karaya, Tragacanth-Harz, Ghatti, Psyllium, Leinsamen, Okra, Xanthan, Gellan, Carboxymethylcellulose, Carboxymethylguar, Carboxymethylstärke und Hyaluronsäure; die sulfatisierten Polysaccharide ausgewählt sind aus Carrageenan, Heparin, Chondroitinsulfat, Dermatansulfat, Heparansulfat, Keratansulfat, Keratan und Cellulosesulfat; und die phosphatierten Polysaccharide ausgewählt sind aus phosphatierter Cellulose, phosphatierter Stärke und phosphatiertem Glucan.

**5.** Als Nahrung aufnehmbare Zusammensetzung, die die im wesentlichen wasserunlöslichen, mit Kationen komplexierten, anionischen Polysaccharide umfaßt, wie in einem der vorhergehenden Ansprüche definiert, die in einem wäßrigen Medium dispergiert sind, worin die als Nahrung aufnehmbare Zusammensetzung eine niedrige Viskosität hat und wärmesterilisierbar ist.

**6.** Als Nahrung aufnehmbare Zusammensetzung gemäß Anspruch 5, die zusätzlich wenigstens eine Zusammensetzung umfaßt, die ausgewählt ist aus Aminosäuren, Peptiden und Proteinen.

**7.** Verwendung eines im wesentlichen wasserunlöslichen, mit Kationen komplexierten, anionischen Polysaccharids gemäß einem der Ansprüche 1 bis 4 zur Herstellung einer als Nahrung aufnehmbaren Zusammensetzung zur Behandlung von Gewichtsverlust eines Tieres, worin eine therapeutisch wirksame Menge der als Nahrung aufnehmbaren Zusammensetzung dem Tier enteral verabreicht wird.

**8.** Verwendung eines im wesentlichen wasserunlöslichen, mit Kationen komplexierten, anionischen Polysaccharids gemäß einem der Ansprüche 1 bis 4 zur Herstellung einer als Nahrung aufnehmbaren Zusammensetzung zur Behandlung von Diarrhoe in einem Tier, worin eine therapeutisch wirksame Menge der als Nahrung aufnehmbaren Zusammensetzung dem Tier enteral verabreicht wird.

**9.** Verwendung gemäß Anspruch 7 oder 8, worin dem Tier ein Teil seines Magen-Darm-Traktes durch ein chirurgisches Verfahren entfernt wurde.

**10.** Verfahren zur Herstellung eines im wesentlichen wasserunlöslichen, wärmesterilisierbaren, mit Kationen komplexierten, anionischen Polysaccharids, wie in einem der Ansprüche 1 bis 4 definiert, welches das Anfeuchten eines im wesentlichen wasserlöslichen, anionischen Polysaccharids mit Wasser auf einen Gesamtfeststoffgehalt von ca. 30 bis 70 % und die Zugabe einer Kationenquelle, gefolgt von Mischen in einem Hochgeschwindigkeitsmischer auf einen Gesamtfeststoffgehalt von ca. 30 bis 70 % umfaßt.

**11.** Niederviskose, wärmesterilisierbare Ernährungszusammensetzung, umfassend: Kohlehydrate, die ca. 1 bis 99 % der Gesamtkalorien liefern; Proteine, Peptide und Aminosäuren, die ca. 1 bis 99 % der Gesamtkalorien liefern; Lipide, die essentielle Fettsäuren enthalten und ca. 1 bis 99 % der Gesamtkalorien liefern; Vitamine, Mineralien;

Wasser; und wenigstens ein im wesentlichen wasserunlösliches, mit Kationen komplexiertes, anionisches Polysaccharid, wie in einem der Ansprüche 1 bis 4 definiert.

**12.** Zusammensetzung, umfassend: Kohlehydrate, die ca. 1 bis 99 % der Gesamtkalorien liefern; Proteine, Peptide und Aminosäuren, die ca. 1 bis 99 % der Gesamtkalorien liefern; Lipide, die essentielle Fettsäuren enthalten und ca. 1 bis 99 % der Gesamtkalorien liefern; Vitamine; Mineralien; und wenigstens ein im wesentliches wasserunlösliches, mit Kationen komplexiertes, anionisches Polysaccharid, wie in einem der Ansprüche 1 bis 4 definiert.

**13.** Nahrungsmittel, umfassend eine Zusammensetzung wie in Anspruch 5 oder 6 definiert.

**14.** Pharmazeutikum, umfassend eine Zusammensetzung wie in Anspruch 5 oder 6 definiert.

## Revendications

**1.** Polysaccharide anionique complexé avec un cation, pratiquement insoluble dans l'eau, ledit cation étant choisi entre des cations de métaux alcalinoterreux, des cations de métaux alcalins, des cations de métaux de transition, des protéines, des aminoacides, des oligopeptides, des polysaccharides cationiques ou à fonctionnalisation amino, des polymères synthétiques cationiques ou à fonctionnalisation amino ou bien leurs associations, ledit polysaccharide anionique complexé avec un cation comprenant des particules sous une forme agglomérée sèche qui se dispersent et se dissocient aisément lors de l'addition à un milieu aqueux en libérant des particules plus petites ayant un diamètre médian de particules inférieur à 100 μm.

**2.** Polysaccharide anionique complexé avec un cation, pratiquement insoluble dans l'eau, suivant la revendication 1, dans lequel ledit cation est choisi entre le calcium, le fer, le magnésium, le zinc, le potassium, le sodium, le cuivre et le manganèse ainsi que leurs mélanges.

**3.** Polysaccharide anionique complexé avec un cation, pratiquement insoluble dans l'eau, suivant la revendication 1 ou la revendication 2, dans lequel ledit polysaccharide anionique est choisi entre des polysaccharides carboxylés, des polysaccharides sulfatés et des polysaccharides phosphatés.

**4.** Polysaccharide anionique complexé avec un cation, pratiquement insoluble dans l'eau, suivant la revendication 3, dans lequel le polysaccharide carboxylé est choisi entre des alginates, des pectines, la gomme arabique, la gomme karaya, la gomme adragante, la gomme ghatti, le polysaccharide du psyllium, le polysaccharide des graines de lin, la gomme okra, la gomme xanthane, la gomme gellane, la carboxyméthylcellulose, la gomme guar carboxyméthylique, le carboxyméthylamidon et l'acide hyaluronique ; le polysaccharide sulfaté est choisi entre la carragénine, l'héparine, le chondroïtine-sulfate, le dermatane-sulfate, l'héparane-sulfate, le kératane-sulfate, le kératane et le sulfate de cellulose ; et le polysaccharide phosphaté est choisi entre la cellulose phosphatée, l'amidon phosphaté et le glucane phosphaté.

**5.** Composition apte à l'ingestion, comprenant le polysaccharide anionique complexé avec un cation, pratiquement insoluble dans l'eau, répondant à la définition suivant l'une quelconque des revendications précédentes, dispersé dans un milieu aqueux, ladite composition apte à l'ingestion ayant une faible viscosité et étant stérilisable par la chaleur.

**6.** Composition apte à l'ingestion suivant la revendication 5, comprenant en outre au moins une composition choisie entre des aminoacides, des peptides et des protéines.

**7.** Utilisation d'un polysaccharide anionique complexé avec un cation, pratiquement insoluble dans l'eau, suivant l'une quelconque des revendications 1 à 4 pour la préparation d'une composition apte à l'ingestion pour le traitement d'une perte de poids chez un animal, dans laquelle une quantité thérapeutiquement efficace de ladite composition apte à l'ingestion est administrée par voie entérale à cet animal.

**8.** Utilisation d'un polysaccharide anionique complexé avec un cation, pratiquement insoluble dans l'eau, suivant' l'une quelconque des revendications 1 à 4 pour la préparation d'une composition apte à l'ingestion pour le traitement de la diarrhée chez un animal, dans lequel une quantité thérapeutiquement efficace de ladite composition apte à l'ingestion est administrée par voie entérale à cet animal.

**9.** Utilisation suivant la revendication 7 ou la revendication 8, dans laquelle l'animal a subi l'ablation d'une partie de son tractus gastro-intestinal par une opération chirurgicale.

10. Procédé pour la préparation d'un polysaccharide anionique complexé avec un cation, pratiquement insoluble dans l'eau, stérilisable par la chaleur, répondant à la définition suivant l'une quelconque des revendications 1 à 4, comprenant le mouillage d'un polysaccharide anionique essentiellement hydrosoluble avec de l'eau à une teneur en matières solides totales d'environ 30 à 70 %, l'addition d'une source de cations puis un mélange dans un mélangeur à forte intensité à une teneur en matières solides totales d'environ 30 à 70 %.

11. Composition nutritionnelle de faible viscosité, stérilisable par la chaleur, comprenant : des glucides fournissant approximativement 1 à 99 % des calories totales ; des protéines, des peptides et des aminoacides fournissant approximativement 1 à 99 % des calories totales ; des lipides contenant des acides gras essentiels, fournissant approximativement 1 à 99 % des calories totales ; des vitamines ; des substances minérales, de l'eau et au moins un polysaccharide anionique complexé avec un cation, pratiquement insoluble dans l'eau, répondant à la définition suivant l'une quelconque des revendications 1 à 4.

12. Composition comprenant : des glucides fournissant approximativement 1 à 99 % des calories totales ; des protéines, des peptides et des aminoacides fournissant approximativement 1 à 99 % des calories totales ; des lipides contenant des acides gras essentiels, fournissant approximativement 1 à 99 % des calories totales ; des vitamines ; des substances minérales et au moins un polysaccharide anionique complexé avec un cation, pratiquement insoluble dans l'eau, répondant à la définition suivant l'une quelconque des revendications 1 à 4.

13. Aliment comprenant une composition répondant à la définition suivant la revendication 5 ou la revendication 6.

14. Composition pharmaceutique comprenant une composition répondant à la définition suivant la revendication 5 ou la revendication 6.